# EUROPEAN PATENT SPECIFICATION

(11) **EP 0 688 336 B1**
(45) Date of publication and mention of the grant of the patent: **28.05.1997**
(21) Application number: 94909098.9
(22) Date of filing: 01.03.1994
(51) Int. Cl.: C07K 5/06, C07K 5/08, C07K 5/10, C07K 7/06, A61K 38/55

(54) **PEPTIDE BORONIC ACID DERIVATIVES HAVING PROTEASE INHIBITING ACTIVITY**
PEPTIDBORONSÄUREDERIVATE MIT PROTEASE INHIBIERENDEN AKTIVITÄT
DERIVES D'ACIDE BORE PEPTIDIQUE A ACTIVITE D'INHIBITION DES PROTEASES

(30) Priority: 03.03.1993 GB 9304289; 01.04.1993 GB 9306822
(43) Date of publication of application: 27.12.1995
(73) Proprietor: Novartis AG, 4058 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: COOK, Nigel Scott, CH-4207 Bretzwil (CH); METTERNICH, Rainer, D-79594 Inzlingen (DE); TAPPARELLI, Carlo, CH-4111 Seltisberg (CH); WIENAND, Anette, D-79423 Heitersheim (DE)
(86) International application number: EP9400595
(87) International publication number: WO9420526

(56) References cited:
- EP-A- 0 145 441
- EP-A- 0 293 881
- EP-A- 0 471 651

## Description

This invention relates to inhibitors of serine proteases involved in the blood coagulation process such as thrombin (factor IIa), factor Xa, factor VIIa, factor XIIa, kallikrein, plasmin, prolyl endopeptidase and Ig AI Protease.

Thrombin, the last enzyme in the coagulation system, cleaves soluble fibrinogen to fibrin, which is then crosslinked and forms an insoluble gel forming the matrix for a thrombus. When a vessel is damaged, the above process is necessary to stop bleeding. Under normal circumstances there is no measurable amount of thrombin present in plasma. Increase of the thrombin concentration can result in formation of clots, which can lead to thromboembolic disease, one of the most common serious medical problems of our time.

Thrombin contributes to haemostatic control by means of several biological reactions. In addition to its primary function, the conversion of fibrinogen to fibrin, thrombin activates Factor XIII, which is responsible for the crosslinking of fibrin. Thrombin also acts by means of a positive feed back mechanism involving the activation of Factors V and VIII, both of which are necessary for formation of thrombin from prothrombin. Thrombin has another essential role: its binding to platelets initiates platelet release and aggregation which is responsible for primary haemostasis.

The reactions of thrombin are further controlled by natural inhibitors in plasma. The most important of these are antithrombin III and heparin. These two compounds have been isolated and are used therapeutically and prophylactically in conditions where there is an imbalance in the haemostatic mechanism with risk of prothrombin activation.

Orally active synthetic thrombin inhibitors would be useful as alternatives to the parenteral administration of these natural inhibitors. Much research in this area has resulted in the synthesis of good inhibitors of thrombin in vitro, but as yet there is no really good candidate for oral therapeutic use. By imitating amino acid sequences of fibrinogen, the important natural substrate of thrombin, several good short peptide substrates for thrombin have been produced. These peptide substrates have also been derivatised to provide inhibitors of the enzyme. Thus, the chromogenic substrates D-Phe-Pro-Arg-pNA and D-Phe-Pip-Arg-PNA mimic the sequence preceding the thrombin cleavage site. The corresponding reversible and irreversible inhibitors, D-Phe-Pro-Arginal and D-Phe-Pro-Arg-CH₂Cl show inhibition in vitro in the 10⁻⁸ M range. Chloromethylketones are generally insufficiently specific to be ideal for therapeutic use, and the peptide aldehyde exemplified above has quite a low LD₅₀ value.

Factor Xa is the coagulation enzyme responsible for the generation of thrombin by limited proteolysis of its zymogen, prothrombin. On a weight for weight basis factor Xa is at least 10 times more thrombogenic in vivo than thrombin. This arises from the fact that factor Xa is one step above thrombin in the amplifying cascade system, so that one molecule of factor Xa can generate many molecules of thrombin from its precursor. Its potency may also arise from the relatively slow removal of factor Xa by the body. Thrombin, unlike factor Xa, is rapidly cleared from circulating blood onto high affinity sites on the vessel wall.

The binding of tissue factor to factor VII represents a key event in initiation of blood coagulation after tissue injury. First it catalyses the activation of factor IX and factor X and secondly, trace concentrations of factor Xa and factor IXa (and concievably other enzymes released from cells after tissue injury) convert zymogen factor VII/tissue factor complexes into highly active factor VIIa/tissue factor complexes.

The central position of factor Xa at the junction of the intrinsic and the extrinsic pathways as well as the crucial role of factor VIIa in initiating both the intrinsic and extrinsic coagulation pathways make factors Xa and VIIa suitable targets for modulating the haemostatic process.

Kallikrein is formed from prekallikrein by the action of factor XII, in the presence of a negatively charged surface. Kallikrein in turn can cleave factor XII to factor XIIa, thereby forming a reciprocal activation system. Factor XIIa is the first enzyme of the intrinsic part of the coagulation system. The significance of the contact system is probably as a surface mediated defence mechanism, and a large scale activation of the system is normally seen during shock or disseminated intravascular coagulation (DIC). The role of kallikrein at this stage is to cleave high molecular weight kininogen with the release of the vasodilator, bradykinin. Bradykinin also causes increased vascular permeability, pain and migration of the neutrophil leucocytes. Inhibitors of kinin formation have been shown to be beneficial in certain types of inflammation, including arthritis and pancreatitis, and may be useful also in the treatment of asthma. The only substance that has attained clinical significance as a kallikrein inhibitor, is aprotinin (Trasylol). Aprotinin is a polypeptide of molecular weight 6,500, and forms a stable complex with proteases, having a binding constant of 10⁻¹⁰-10⁻¹³ M.

Fibrinolysis is the process of enzymatic dissolution of fibrinogen and fibrin clots. Plasma contains a protein, plasminogen, which under the influence of various activators is converted to plasmin, a proteolytic enzyme, the activity of which resembles that of trypsin. Plasmin breaks down fibrinogen and fibrin to fibrin/fibrinogen degradation products.

Under normal conditions, the fibrinolysis system is in balance with the coagulation system. Small thrombi formed in the blood stream are dissolved enzymatically and the circulation through the vessels is restored by the activation of the fibrinolytic system in the body. If the fibrinolytic activity is too high, it may cause or prolong bleeding. The activity can be inhibited by natural inhibitors in the blood.

Prolyl endopeptidase cleaves peptide bonds on the carboxyl side of proline residues within a peptide chain. It is a serine protease which readily degrades a number of neuropeptides including substance P, neurotensin, thyrotropin-releasing hormone and luteinizing hormone-releasing hormone and which has been associated with the ability of cells to produce interleukin 2 (IL-2). The enzyme is inhibited by benzyloxycarbonyl-prolyl--prolinal with a Ki of 14 nM. Although little is known about the physiological role of prolyl endopeptidase, it may play a prominent role in the regulation of the biological activities of various neuropeptides.

The Ig A proteinase-catalyzed cleavage of Ig A, the predominant form of antibody which comprises the first line of defence against infection, separates the Fc from the antigen-binding Fab regions of the molecule. Such cleavage would be expected to impair or abolish its antimicrobial activity. All Ig A proteinases identified thus for cleave after a proline residue within the hinge region of human Ig A. Peptide prolyl-boronic acids inhibit Ig A 1 proteinases from Neisseria gonorrhoea and Hemophilus influenzae indicating these enzymes belong to the serine protease family, of proteclytic enzymes.

The multiple roles played by thrombin in a variety of physiological processes which have been associated with pathological disorders such as cancer, inflammation and neuronal activity, suggest a potential use of thrombin inhibitors in several indications not strictly related to the cardiovascular system.

Many tumor cells have been shown to elicit procoagulant activity associated with the generation of thrombin. As a consequence, local fibrin deposition and coagulation occur which are thought to be important for tumor growth. Additionally, due to its effects on endothelial cells, thrombin may facilitate the extravasation of tumor cells during metastasis. Hence, thrombin inhibitors may prove beneficial not only in the treatment of certain cancers but also in reducing the hypercoagulability frequently observed in patients during therapy with chemotherapeutic agents.

Thrombin activation of endothelial cells induces a number of pro-inflammatory changes such as synthesis and release of interleukin-1, prostaglandins and platelet-activating factor. Additionally, thrombin induces the exposure of CD62 (alternatively known as GMP-140) and CD63, two adhesion molecules responsible for the adhesion of leukocytes to the endothelial surface. Thrombin also increases vascular permeability of proteins involving neutrophils, and cleaves the interleukin-8-precursor protein, a peptide believed to be involved in respiratory disorders, rheumatoid arthritis and ulcerative colitis.

Thrombin's involvement in all these pro-inflammatory processes make thrombin inhibitors a potential target for use in the therapeutic treatment of inflammation-related pathological disorders.

The activity of the protease nexin-1, a modulator of nerve growth and a specific natural thrombin antagonist, is markedly and specifically decreased in patients with Alzheimer's disease. This, together with the observation that thrombin-like activity was increased in the brains of Alzheimer's patients, suggests that thrombin inhibitors may have potential for limiting or reversing neuronal pathological changes associated with hyperactivity of thrombin or related serine proteases.

Boronic acids have been studied as inhibitors of varieus serine esterases and proteases. The first boronic acid-containing amino acid analog to be used as a protease inhibitor was the boronic acid analog of N-acetyl L-phenylalanine, which was used as an inhibitor of chymotrypsin and subtilisin. Peptide boronic acids have been used as inhibitors of chymotrypsin, subtilisin, and elastases.

The interaction of boronic acids with proteases in biological systems is known and various simple boronic acids have been shown to be sufficiently non-toxic for use in humans. Peptide boronic acid inhibitors of elastase have recently been used in animal trials in relation to emphysema. Unlike the peptide chloromethylketones, there was no toxicity reported at biologically effective dosage levels.

European Patent Application 293 881 (Dupont) describes the preparation of peptides comprising C-terminal boronic acid derivatives of lysine, ornithine and arginine, and their use as inhibitors of trypsin-like serine proteases. The other amino acids of the peptides are all either the D- or the L- forms of the 20 naturally-occurring amino acids.

It has been found that compounds, having superior properties as inhibitors of trypsin-like serine proteases, are obtained when the peptide contains at least one unnatural α-amino acid having a hydrophobic side chain. Compounds of this latter type are described in our copending application, published European patent application EP O 471 651, being compounds of formula I 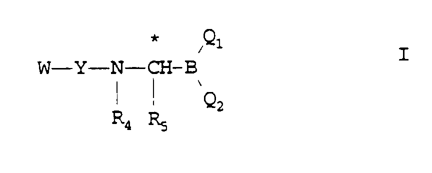 wherein W = H or an N-protecting group;
Y is a sequence of n amino acids selected such that the n+1 amino acid peptide Y-Lys or Y-Arg has an affinity for the active site of a trypsin-like protease, where n is an integer from 1 to 10, and in which at least one amino acid is an unnatural amino acid having a hydrophobic side chain;
Q₁ and Q₂, which may be the same or different, are selected from -OH, -COR₁, -CONR₁R₂, -NR₁R₂, and -OR₃, or Q₁ and Q₂ taken together form a diol residue; R₁, R₂ and R₃, which may be the same or different, are C₁₋₁₀ alkyl, C₆₋₁₀ aryl, C₆₋₁₀ aralkyl, or phenyl substituted by up to three groups selected from C₁₋₄ alkyl. halogen and C₁₋₄ alkoxy;
R₄ is hydrogen or C₁₋₁₀alkyl;
R₅ is a group -A-X
   wherein A is -(CH₂)_{z}- in which z is 2,3,4 or 5; -CH(CH₃)-(CH₂)₂-; -CH₂-CH(CH₃)-CH₂-; -(CH₂)₂-CH(CH₃)-; -(CH₂)₂-C(CH₂)₂-; -CH(CH₃)-(CH₂)₃-; -CH₂-CH(CH₃)-(CH₂)₂-; -CH₂-CH₂-CH(CH₃)-CH₂; (CH₂)₃-CH( CH₃)-; -(CH₂)₃-C(CH₃)₂; C₆₋₁₀aryl, C₆₋₁₀aralkyl
   and wherein X is -NH₂, -NH-C(NH)-NH₂, -S-C(NH)-NH₂, -N₃, C₁₋₄alkoxy, C₁₋₄alkylthio or -Si(CH₃)₃ or R₄ and R₅ together form a trimethylene group and the asymmetric carbon atom marked ∗ may have the D- or L-configuration or represent any mixture of these.

An unnatural amino acid is defined as any amino acid other than D- or L- Ala, Arg, Asp, Cys, Gln, Glu, Gly, His, Ile, Leu, Lys, Met, Phe, Pro, Ser, Thr, Trp, Tyr, or Val.

It has now been found that further improvements may be made to this latter type of compound by appropriate choice of the residue R₅.

Accordingly the present invention provides compounds of formula I and salts thereof
wherein W, R₄, Q₁ and Q₂ are as defined in EP 0 471 651, Y is a sequence of two amino acids of which the N-terminal amino acid is an unnatural amino acid having a hydrophobic side chain and the other amino acid is L-proline, the unnatural amino acid being selected such that the amino acid peptide Y-Lys or Y-Arg has an affinity for the active site of a trypsin-like protease, and
R₅ is a group -A-X
   wherein A is -(CH₂)_{z}- in which z is 2,3,4 or 5; -CH(CH₃)-(CH₂)₂-; -CH₂-CH(CH₃)-CH₂-; -(CH₂)₂-CH(CH₃)-; -(CH₂)₂-O(CH₃)₂-; -CH(CH₃)-(CH₂)₃-; -CH₂-CH(CH₃)-(CH₂)₂-; -CH₂-CH₂-CH(CH₃)-CH₂; -(CH₂)₂C(CH₃)₂-CH₂-; -(CH₂)₃-CH(CH₃)-; -(CH₂)₃-C(CH₃)₂; -(CH₂)₃CH(CH₃)CH₂-; C₆₋₁₀aryl, C₆₋₁₀aralkyl
   and wherein X is OH, SH, NR₆R₇ or phenyl,
   wherein R₆ is H or C₁₋₁₀alkyl, and R₇ is C₁₋₁₀alkyl, -CO-R₈, CS-R₈, or

   SO₂-R₈

   wherein R₈ is H or C₁₋₁₀alkyl, C₁₋₁₀alkoxy, C₆₋₁₀aryl, C₆₋₁₀aralkyl optionally substituted by up to three groups selected from halogen, OH, C₁₋₄alkoxy, C(NH)R₉, NR₁₀R₁₁ and C(O)OR₆ (wherein R₆ is as defined above),
   wherein R₉ is C₁₋₃alkyl or N(CH₃)₂ and wherein R₁₀ and R₁₁ (which may be the same or different) are H or C₁₋₁₀alkyl,
and the asymmetric carbon atom marked ∗ may have the D- or L-configuration, and the compounds may comprise any mixture of these.

Unnatural amino acid has the same meaning as in EP 0 471 651.

Preferably the N-protecting group W is of formula H(CH₂CH₂O)ₚ- where p = 3-30; R₁₂CO-; R₁₃OCO- or R₁₄SO₂
in which R₁₂ is C₁₋₆ alkyl or H(CH₂-O-CH₂)_{P} wherein p is as defined above; R₁₃ is C₁₋₆ alkyl, phenyl, benzyl or naphthyl; and R₁₄ is phenyl, naphthyl or C₁₋₄ alkylphenyl; of which R₁₃OCO- is particularly preferred. The most preferred protecting groups are those of formula R₁₃'OCO- in which R₁₃' is tert- butyl (designated Boc), and in which R₁₃, is benzyl (designated Z).

Preferably R₅ is R₅' where R₅' is -(CH₂)_{z'}-X', in which X' is OH, phenyl, NH-CO-R₈', NH-CS-R₈', NHSO₂R₈' or NR₁₀'R₁₁',
wherein R₈' is H, or C₁₋₃alkyl or C₁₋₃alkoxy and wherein R₁₀' and R₁₁' which may be the same or different are H or C₁₋₃alkyl optionally substituted with up to 3 halogen atoms, and z' is 2,3 or 4.

More preferably R₅ is R₅" where P₋₅" is -(CH₋₂)_{z'}-X", in which X" is OH, C₆H₅ C₆H₄Cl, C₆H₄N(Me)₂, NH-CO-CH₃, NH-CHO, NH-CS-NHCH₃, NH-CO-CH(CH₃)₂, NH-CO-CH₂-CH_{3,} NH-CHS, NH-CO-CH₂Cl, NH-CO-OCH₃ and z' is as defined above.

Preferred compounds are those of formula Ia 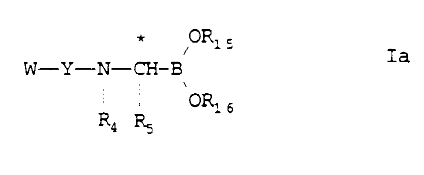 wherein: W, Y, R₄ and R₅ are as defined above and R₁₅ and R₁₆ represent the residue of a dihydroxy compound.

Useful examples of dihydroxy compounds are, 2,3-butanediol; catechol; 2,3-dimethyl; 2',3'-butandiol; cyclohexanediol; ethylene giycol; 1,2-hexanediol; 2,3-hexanediol; diethanolamine or aliphatic or aromatic compounds having hydroxy groups that are substituted on adjacent carbon atoms or on carbon atoms substituted by another carbon atoms.

Particularly preferred are those compounds in which Q₁ and Q₂ taken together represent the group OPin of formula a) or the group of formula b) 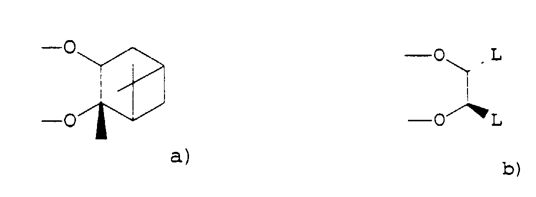 in which L is a C₁₋₄alkyl group, especially methyl, i-Pr, n-Pr or n-Bu.

The unnatural amino acid may be an alkylated natural amino acid such as O-alkyl or S-alkyl-cysteine. However, preferred unnatural amino acids are of formula II 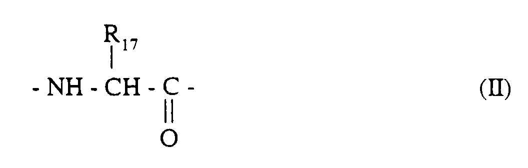 in which R₁₇ is a hydrophobic group consisting of an alicyclic group having at least two rings and no polar substituents linked to the amino acid either directly or through a methylene group; or a methylene group linked to an aromatic group optionally substituted by a polar group, or to a tert. butyl or trimethylsilyl group. Preferably R₁₇ is R₁₇' where R₁₇' is a group of formulae c), d), e), f), g), h) or i)

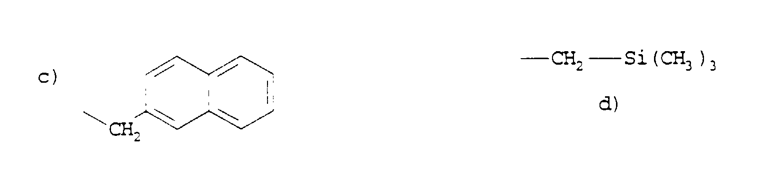

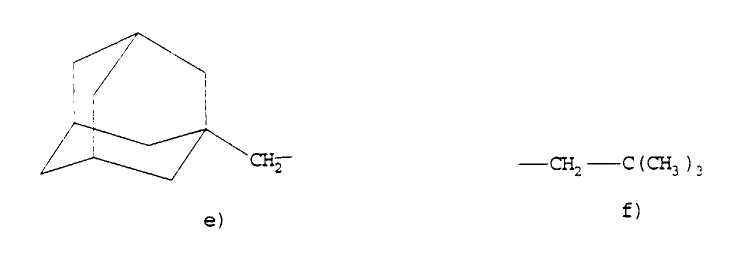

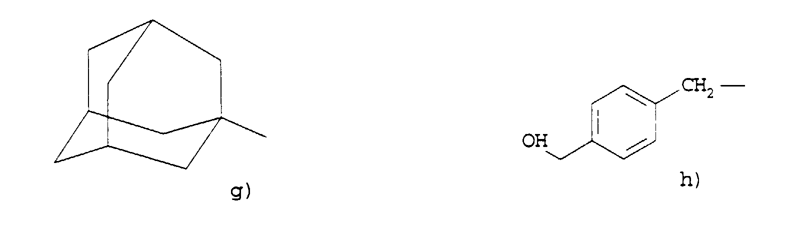

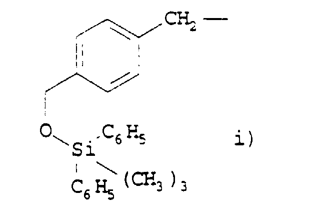

The unnatural amino acids of formula II may be in D- or L- form or any mixture of these, but are preferably in D-form.

More preferred compounds have the formula I' 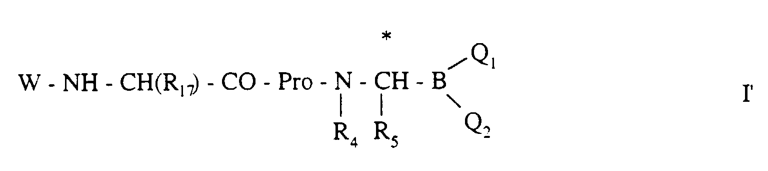 in which W, R₄, R₅, R₁₇, Q₁, and Q₂ are as defined above.

Particularly preferred compounds are those of formula I" 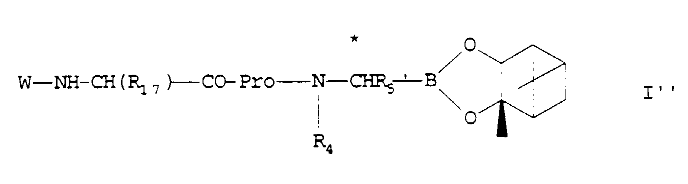 in which W, R₄, R₅' and R₁₇ are as defined above.

The most preferred compounds are the compound of formula III, 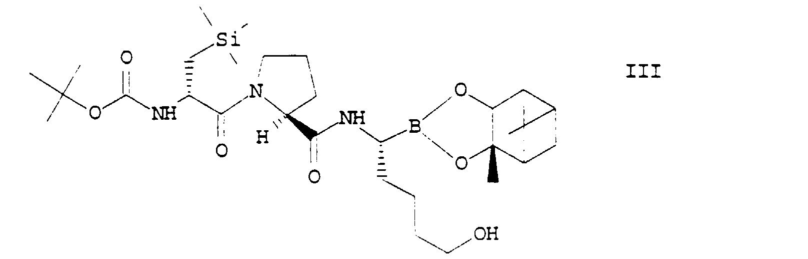 the compound of formula IV, 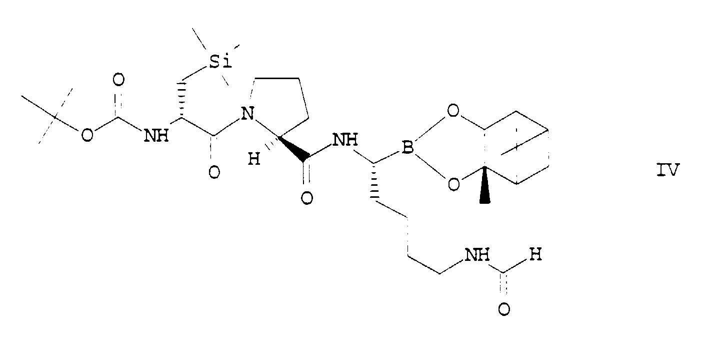 the compound of formula V 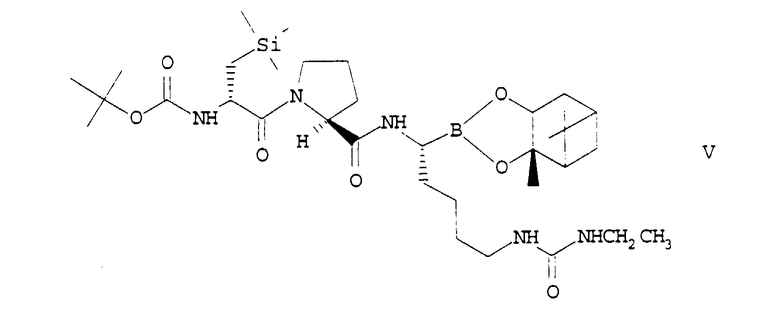 and the compound of formula VI

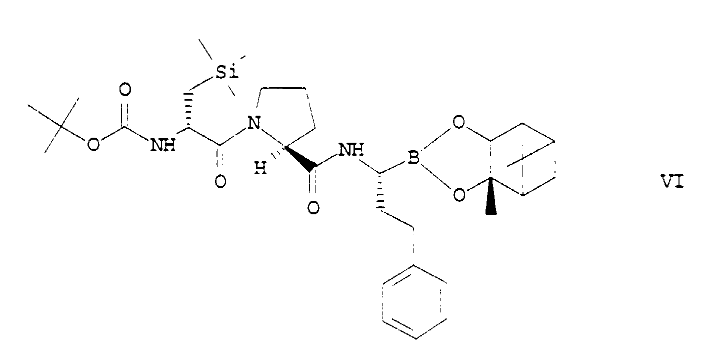

A peptide is considered to have an affinity for the active site of trypsin-like proteases if the dissociation constant of the peptide-protease complex has a value of 10⁻⁵ M or lower.

The compounds of formula I in which X is NHCHO or NHCOalkyl may be prepared by reacting a compound of formula I in which X is -NH₂ with an active form of the corresponding acid.

The compounds of formula I in which X is NHCOalkoxy may be prepared by treating a compound of formula I in which X is -NH₂ with N,O-Bis (trimethylsilyl) acetamide followed by addition of an active form of the corresponding ester.

The compounds of formula I in which X is NHC(S)NHalkyl may be prepared by reacting a compound of formula I in which X is -NH₂ with the corresponding alkyl isothiocyanate in an organic solvent.

Compounds of formula I in which X is -NH₂ may in turn be produced by hydrogenation of a compound of formula I in which X is -N₃. Hydrogenation may be carried out under standard conditions using for example a Pd/C catalyst.

Compounds of formula I in which X is -N₃ may be produced by reaction of a compound of formula VI 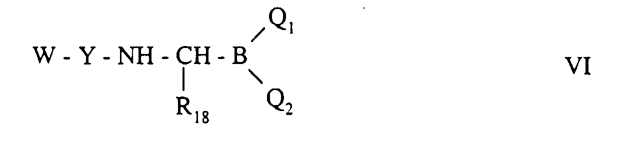 in which W, Y, Q₁ and Q₂ are defined above and R₁₈ is -A-Br wherein A is as defined above with sodium azide in a polar aprotic solvent such as dimethyl sulphoxide.

The compounds of formula I in which X is -NHCHS may be prepared by reacting the compound of formula I in which X is NHCHO with Laweson reagent in an organic solvent such as toluol.

The compounds of formula I in which X is optionally substituted phenyl may be obtained by the reaction of a compound of formula VII 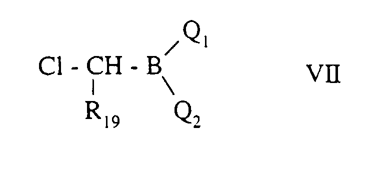 in which Q₁ and Q₂ are defined above and R₁₉ is -A-phenyl (optionally substituted) wherein A is as defined above, with LiN[Si(CH₃)₃]₂ followed by hydrolysis with an excess of acid and coupling with a protected peptide of formula VIII 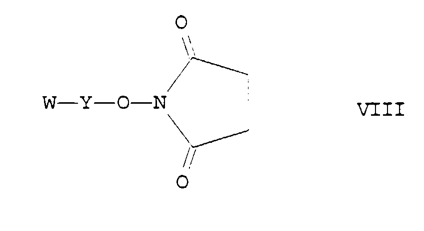 wherein W and Y are as defined above

The reaction is preferably carried out in a dry aprotic polar solvent, for example tetrahydrofuran, at a temperature between -78°C and room temperature.

The intermediates of formula VII may be obtained by the method of Matteson et al, Organometallics 3 1284-8 (1984).

The protected peptide of formula VIII may be prepared by methods which are conventional in peptide chemistry, starting from the desired unnatural amino acid. Such amino acids are either commercially available (for example the amino acid in which R₁₆ is the group c) or may be prepared by methods analogous to those described in the literature, e.g. Angew. Chem. 93, 793 (1981) and J. Am. Chem. Soc. 109, 6881 (1987).

The compounds of fomula I in which X is OH may be obtained by reacting a compound of formula I in which X is -OSi(CH₃)₂ C(CH₃)₃ with a desilylating agent, such as tetrabutylammonium-fluoride.

The compounds of formula I are useful as inhibitors of trypsin-like proteases and may be used in vitro for diagnostic and mechanistic studies of these enzymes. Furthermore, because of their inhibitory action they are indicated for use in the prevention or treatment of diseases caused by an excess of an enzyme in a regulatory system, for example control of coagulation and fibrinolysis.

The compounds of the invention have trypsin-like serine protease inhibiting properties, as indicated in the following test methods:

### a) Enzyme inhibition kinetics

Test substance is dissolved in cremophor/ethanol (1:1) or DMSO and diluted with distilled water to yield a 1 mM stock solution. Further dilutions are made into the assay buffer (100 mM sodium phosphate buffer, pH 7.4, containing 100 mM NaCl and 0.1 % bovine serum albumin). Kinetic assay are performed using a 96-microwell plate; each well contains 50 µl substrate, 100 µl test compound and 100 µl enzyme in buffer. Final concentrations of substrate and enzyme are as follows: 160 pM α-thrombin and 100 µl Pefachrom TH (Kₘ = 6.9 µM), 800 pM human plasmin and 200 µM Pefachrome PL (Kₘ = 66.4 µM), and 260 pM bovine pancreatic trypsin and 500 µM Pefachrome TRY (Kₘ = 167.7 µM). Assays are initiated by adding enzyme to solutions containing the substance to be tested and substrate. The release of p-nitroaniline from the hydrolysis of the substrate is followed for 30 min by measuring the increase in optical density at 405 nm with a Thermomax microwell kinetic reader (Molecular Devices, Menlo Park CA, USA). When the inhibited steady-state rate is achieved rapidly, the inhibition constant (Kᵢ) is determined by fitting the data by weighted linear regression to the Dixon equation (Biochem. J. 1953, 55:170-171). For slow-, tight-binding inhibitors, the mechanism of inhibition may be described by the following scheme: 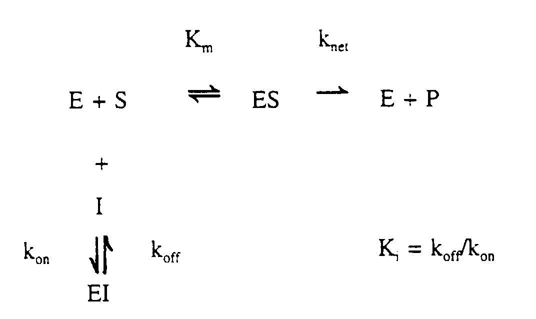 where E, S, P, and I are the enzyme, substrate, product (p-nitroaniline) and inhibiting substance to be tested, respectively, and kₒₙ and k_{off} are the association and dissociaton rate constants for the inhibition (Tapparelli et al., J. Biol. Chem., 268 (1993), 4734-4741). Progress-curve data for the formation of p-nitroaniline in the presence of different concentrations of inhibitor are fitted by nonlinear regression to the equation for the mechanism presented in the scheme (Morrison and Walsh, Adv. Enzymol. Relat. Areas Mol. Biol. 1988, 61:201-301). These analyses yield estimates for the apparent values of kₒₙ, k_{off} and Kᵢ which are corrected for the presence of substrate as described by Morrison and Walsh to give the true values. Under the described experimental conditions a Kᵢ of 13 nM is observed for the compound of Example 1.

### b) In vitro coagulation assays

In vitro coagulation assays are performed with pooled citrated human plasma. Substance to be tested or solvent are incubated with plasma for 10 min at 37°C prior to assay. Thrombin time(TT) determinations are performed at a final thrombin concentration of 5 U/ml. Activated partial thromboplastin time (APTT) is determined by incubating 0.1 ml plasma ± substance to be tested with 0.1 ml purified soya bean phospholipid in ellagic acid (Actin-FS) for 4 min at 37°C and followed by the addition of 0.1 ml CaCl₂ (50mM). In this test method, compounds of the invention significantly increase TT and APTT at a concentration of from 0.1 µM to 0.5 µM. With the compound of Example 1, prolongation of TT above 300 sec is achieved at 1.8 µM, elevation of APTT to 2x the control value is achieved at 4.4 µM.

### c) In vitro platelet aggregation assay

Washed human platelets are prepared by a modification of the method of Ardlie (Br. J. Pharmacol. 1970, 19:7-17). Washed platelet suspension (0.46 ml) is kept at 37°C and stirred at 1100 rpm. After the addition of the substance to be tested or solvent. the platelets are maintained for 2 min before aggregation is induced by 1 nM human thrombin. The extent of platelet aggregation is quantitated by the maximum aggregation amplitude and inhibition of aggregation rate observed in the absence of inhibitor. In this test method, compounds of the invention significantly inhibit platelet aggregation at a low nM concentration range. The compound of Example 1 has an IC₅₀ value of 3.8 nM.

Those compounds of the invention which are thrombin or factor Xa inhibitors have anti-thrombogenic properties and may be employed when an anti-thrombogenic agent is needed. Generally, these compounds may be administered orally or parenterally to a host to obtain an anti-thrombogenic effect. In the case of larger mammals such as humans, the compounds may be administered alone or in combination with pharmaceutical carriers or diluents at a dose of from 0.02 to 15 mg/Kg of body weight and preferably 1-10 mg/Kg to obtain the anti-thrombogenic effect, and may be given as a single dose or in multiple doses or as a sustained release formulation. When an extracorporeal blood loop is to be established for a patient, 0.1-1 mg/Kg may be administered intravenously. For use with whole blood from 1-10 mg per liter may be provided to prevent coagulation. Pharmaceutical diluents are well known and include sugars, starches and water which may be used to make tablets, capsules, injectable solutions and the like. The compounds of the invention may be added to blood for the purpose of preventing coagulation of the blood in blood collection or distribution containers, tubing or implantable devices which come into contact with blood.

In the arterio-venous shunt thrombosis model in the rat, a modification of the model described by Butler et al. (Blood Coag. Fibrinol., Vol. 3, (1992), page 155), the compounds of the invention prevent thrombus formation in a dose dependent manner. Using the compound of Example 1, thrombus formation is inhibited by 21% at a dose of 0.3 mg/kg i.v. and blocked by a dose of 3 mg/kg i.v.

Also, in view of their trypsin-like serinc protease inhibition activity, the compounds of formula I are indicated for use for inhibiting vascular remodelling (proliferation, migration) following venous or arterial surgery or other forms of vascular damage.

Percutaneous transluminal coronary angioplasty (PTCA) has become a common surgical procedure for the treatment of atherosclerotic plaques occluding coronary arteries. Angioplasty is also performed, albeit to a lesser extent, on peripheral vessels such as the femoral and renal arteries. The splitting/compression of an atherosclerotic plaque and stretching of the arterial wall during balloon catheter angioplasty additionally results in injury to (or removal of) the endothelial cells and possibly also damage to the underlying smooth muscle cells. This accounts for the primary complications of PTCA, acute reocclusion (build up of an occlusive platelet thrombus on the exposed thrombogenic vessel surface) and chronic restenosis (primarily due to proliferation and migration of smooth muscle cells). Acute restenosis. occuring in ∼5% of patients within 24 hours after PTCA, is life threatening and must be surgically dealt with immediately. Chronic restenosis, diagnosed 3-6 months post-surgery in around 30% of patients, leads to chest pain but is only life threatening if the occlusion is 100% (giving rise to myocardial infarction if the plaque should rupture).

The possible involvement of thrombin in the vascular response to injury has recently come under scrutiny. By its action on platelets, thrombin could be involved in the early platelet recruitement and activation at the site of damage, and by virtue of its mitogenic action on smooth muscle cells, thrombin could also contribute to intimal thickening. Thrombin concentrations are likely to be elevated at sites of vascular injury because of continued activation of the coagulation pathways in the absence of a normal endothelial layer and also due to release of clot-bound thrombin during the thrombolytic process. Additionally, thrombin becomes bound to the extracellular matrix where it can remain active, and protected from the inhibitory effect of circulating antithrombins, for prolonged periods.

The interaction of platelets with a severly injured vessel wall appears in part to be dependent on local thrombin generation.

Whilst the accumulated evidence points to thrombin inhibition being a potentially attractive principle to limit restenosis following balloon angieplasty available agents all have the disadvantage of needing to be administered parenterally (or locally) in order to obtain sufficient local concentrations to demonstrate an effect, or other side effects may be limiting.

The compounds of the invention may be used to inhibit vascular remodelling at doses similar to those at which they are used as anti-thrombogenic agents.

Advantageously the compounds of the invention are orally active, have rapid onset of activity and low toxicity. In addition, these compounds may have particular utility in the treatment of individuals who are hypersensitive to compounds such as heparin.

In the following examples, the symbols have the following meanings:
- Z =: benzyloxycarbonyl
- Boc =: t-butyloxycarbonyl
- Ac =: acetyl
- MeOH =: methyl alcohol
- EtOAc =: ethyl acetate
- DMF =: dimethyl formamide
- DCC =: dicyclohexylcarbodiimide
- HONSu =: N-hydroxy-succinimide
- OPin =: pinanediol
- THF =: tetrahydrofuran
- n-Bu =: n-butyl
- Np =: p-nitrophenyl
- TLC =: thin layer chromatography
- Bzl =: benzyl
- TMSal =: trimethylsilylalanine
- BoroPro-OPin =: analog of proline in which the -COOH group is replaced by B-0Pin
- BoroLys =: -NH-CH-(CH₂-CH₂-CH₂-CH₂-NH₂)B-

### Example 1

### Boc-D-TMSal-Pro-NH-CH[(CH₂)₄OH]B-OPin

### A. Boc-D-TMSal-OH

21.5 g (113.7 mmol) D-TMSal ethyl ester. prepared according to the procedure given in Angew. Chem. **93**, 793 (1981), is dissolved in CH₂Cl₂ and a solution of an excess of Boc₂O in CH₂Cl₂ is added. After 15 h at room temperature, 500 ml of ice-cold 0.25 N hydrochloric acid is added. The organic layer is washed with 5 % NaHCO₃ and brine, then dried over Na₂SO₄ and concentrated in vacuo.

The crude material (colourless oil) is used directly in the saponification step. It is dissolved in methanol, cooled to 0°C, mixed with 510 ml of 1 N NaOH and stirred at 0°C for 3 h. After acidification to pH 1 with 1 N HCl the mixture is extracted several times with ether. The organic layers are combined, washed with brine, dried over Na₂SO₄ and concentrated in vacuo. The product (29.7 g oil) is used in the next step without further purification.

### B. Boc-D-TMSal-Pro-ONSu

29.7 g (113.7 mmol) Boc-D-TMSal-OH and 19.0 g (136.3 mmol) p-nitrophenol are dissolved in EtOAc. After cooling to 0°C, 23.4 g (113.6 mmol) DCC is added and the mixture is stirred for 1 h at 0°C and then for 15 h at room temperature. The precipitate is filtered off and washed with EtOAc and the filtrate is concentrated in vacuo. The resulting oil is purified by flash chromatography (9:1, hexane/EtOAc) to give the desired Boc-D-TMSal-ONp as white crystals.

51.6 g (113.7 mmol) Boc-D-TMSal-ONp is dissolved in THF and an aqueous solution of equimolar amounts of proline and Et₃N is added. After 20 h at room temperature, the THF is removed in vacuo and the aqueous residue is diluted with water and then extracted several times with EtOAc. The pH of the aqueous layer is adjusted to 3 by adding 10 % citric acid. The resulting oily product is extracted several times with EtOAc. The combined organic layers are washed with brine, dried over Na₂SO₄ and concentrated in vacuo. The colourless oil is recrystallized from ether/hexane to give the dipeptide Boc-D-TMSal-Pro-OH as a white crystalline compound, mp: 176°C.

26.0 g (72.5 mmol) of the resulting dipeptide is dissolved in EtOAc. After cooling to 0°C, 9.8 g (85.5 mmol) HONSu and 14.9 g (72.3 mmol) DCC are added. The mixture is stirred for 3 h at 0°C and then for an additional 15 h at room temperature. The mixture is recooled to 0°C, the dicyclohexylurea is filtered off and washed several times with EtOAc. The filtrate is washed with aqueous 0.1 M Na₂CO₃ and then with aqueous 2 % KHSO₄. After drying over Na₂SO₄ and concentration in vacuo, Boc-D-TMSal-Pro-ONSu is obtained as a white foam.

### C. (+)-Pinanediol-(S)-1-chloro-5-t-butyldimethylsiloxy-pentane-1-boronate

10.5 ml (120 mmol) 3-Butene-1-ol, 22.3 g (144 mmol) *t*-butyldimethylsilylchloride and 20.4 g (300 mmol) imidazole are dissolved in 60 ml DMF and stirred overnight at 35°C. The two layers are separated and the product layer is washed with 2 N tartaric acid, water and brine. After drying over Na₂SO₄ and concentration in vacuo, 1-*t*-butyldimethylsiloxy-3-butene is obtained as colourless oil.

7.45 g (40 mmol) of this product is stirred for 24 h with 5.1 g (40 mmol) catecholborane at 125°C, yielding a yellow-brown oil. This oil is added to 6.9 g (40 mmol) (+)-pinanediol in 30 ml THF and stirred overnight. After flash chromatography (95:9, hexane/EtOAc) (+)-pinanediol-4-*t*-buty]dimethylsiloxy-butane-1-boronate is obtained.

Over a 20 min period 17 ml (27.0 mmol) BuLi (1.6 M in hexane) is added to a precooled (-100°C) solution of 2.8 ml CH₂Cl₂ and 49 ml THF. After stirring for 15 min at 100°C, 9.0 g (24.5 mmol) of the pinanediolboronate product obtained above in 24 ml THF is added over a 20 min period. The reaction mixture is again stirred for 15 min at -100°C, after which 1.24 g (12.3 mmol) ZnCl₂ (solid) is added. The reaction mixture is allowed to warm up to room temperature overnight. After concentration in vacuo the residue is dissolved in ether/H₂O. The organic layer is dried over Na₂SO₄ and concentrated in vacuo. (+)-pinanediol-(*S*)-1-chloro-5-*t*-butyldimethylsiloxy-butane-1-boronate is obtained as yellow-orange oil.

### D. Boc-D-TMSal-Pro-NH-CH[(CH₂)₄OSi(t-Bu)Me₂]B-OPin

12.8 ml (20.4 mmol) Butyllithium (1.6 M in hexane) is added to a precooled (-78°C) solution of 4.3 ml (20.4 mmol) hexamethyldisilazane in 30 ml THF. The reaction mixture is stirred for 1 h at room temperature, then cooled down to -78°C again. 8.5 g (20.4 mmol) (+)-pinanediol-(*S*)-1-chloro-5-*t*-butyldimethylsiloxy-butane-1-boronate in THF is added. After stirring for 1 h at -78°C, the solution is allowed to warm overnight to room temperature. After recooling to -78°C 3 mol equivalents of HCl in dioxane are added. The mixture is stirred for 1 h at -78°C, then for 2 h at room temperature. After cooling down to -20°C, a solution of 9.28 g (40.8 mmol) of the active ester of Example 1, step B in CH₂Cl₂ is added, followed by the addition of 5.67 ml (40.8 mmol) triethylamine. The mixture is stirred for 1 h at -20°C and for 2 h at room temperature. then filtered. After concentration in vacuo, the residue is dissolved in ether/H₂O. The organic layer is dried over Na₂SO₄ and concentrated in vacuo. After flash chromatography (6:4, hexane/EtOAc), the desired product is obtained; [α]_{D}²⁰ = - 45.6° (c = 0.5 in MeOH); MH⁺ = 736.

### E. Boc-D-TMSal-Pro-NH-CH[(CH₂)₄OH]B-OPin

600 mg (0.816 mmol) of the siloxy compound obtained in step D of Example 1 is dissolved in THF and 514 mg (1.62 mmol) tetrabutylammonium fluoride is added. After stirring overnight at ambient temperature, the mixture is concentrated in vacuo and dissolved in ether/H₂O. The organic layer is dried over Na₂SO₄ and concentrated in vacuo. The side product is removed with EtOAc on silicagel, then the product is eluated with EtOAc/EtOH (9:1), yielding the title product as a white foam; [α]_{D}²⁰ = - 72.4° (c = 0.5 in MeOH); MH⁺ = 622.

### Example 2-4

### Boc-D-TMSal-Pro-NH-CH[(CH₂)₄NHCHO]B-OPin

### A)(+)Pimanediol-(S)-1-chloro-5-bromo-boronate

20.8 ml (203.3 mmol) 4-Bromo-1-butene is reacted with 924.4 g (203.3 mmol) catecholborane at 100°C over 16 h. The crude product is distilled in vacuo to give 4-bromo-butane-1-boronate as a white crystalline product.

27.7 g (163 mmol) (+)-Pinandiol is dissolved in THF and 41.6 g (163 mmol) of 4-bromo-butane-1-boronate as synthesized above is added. After 1 h at room temperatur, the THF is removed in vacuo and the residue is purified by flash chromatography (9:1, hexane/EtOAc) to give (+)-pinanediol-4-bromo-butane-1-boronate as a colourless oil.

The desired (+)-pinanediol-(S)-1-chloro-5-bromo-pentane-1-boronate is prepared according to the procedure given in Organometallics 3, 1284 (1984). 9.8 ml CH₂Cl₂ in THF is cooled to -100°C and 71.6 ml (114.5 mmol) n-butyllithium (1.6 M in hexane) is added over 20 min. After 15 min at -100°C, a cold (-78°C) solution of 32.8 g (104.1 mmol) (+)-pinanediol-5-bromo-pentane-1-boronate in THF is added. After an additional 1 h at -100°C, 7.1 g (52.0 mmol) anhydrous ZnCl₂ in THF is added. After an additional 15 min at -100°C, the reaction mixture is warmed to room temperature. The solvent is removed in vacuo, the residue diluted with hexane/water and extracted several times with hexane. After drying over Na₂SO₄ and removal of the solvent in vacuo, (+)-pinanediol-(S)-1-chloro-5-bromo-pentane-1-boronate is obtained as a yellow oil which is used directly in the next step without further purification.

### B) Boc-D-TMSal-Pro-NH-CH[(CH₂)₄Br]B-OPin

A solution of 65.2 ml (66.2 mmol) LiN(SiMe₃)₂ (1 M in THF) in THF is cooled to -78°C. 23.7 g (65.2 mmol) α-Chloro-boronate of Example 2, step A in THF is added. After stirring for 1 h at -78°C, the mixture is stirred for 15 h at room temperature.

After this period, the reaction mixture is recooled to -78°C. 29.8 ml (6.56 N solution, 196 mmol) HCl in dioxane is added and the solution stirred for 45 min at - 78°C and then for 2 h at room temperature. The mixture is cooled to -15°C, 29.7 g (65.2 mmol) Boc-TMSal-Pro-ONSu of Example 1, step B in CH₂Cl₂ is added, followed by 18.1 ml (130.4 mmol) triethylamine to start the coupling reaction. After stirring at - 15°C for 1 h, the mixture is stirred for 2 h at room temperature. The mixture is then filtered over Hyflo and concentrated in vacuo. The residue is diluted with ether/water and extracted several times with ether. After drying over Na₂SO₄ and concentration in vacuo, the desired Boc-D-TMSal-Pro-NH-CH[(CH₂)₄Br]B-OPin is obtained as white crystals from ether/hexane, mp: 74°C.

### C) Boc-D-TMSal-Pro-NH-CH[(CH₂)₄N₃]B-OPin

33.3 g (48.6 mmol) of the product of Example 2, step B is dissolved in DMSO and 6.3 g (97.2 mmol) sodium azide is added. The mixture is stirred for 6 h at room temperature. Ether/ice water is added, and the resulting oil is crystallized to give Boc-D-TMSal-Pro-NH-CH[(CH₂)₄N₃]B-OPin as a white crystalline compound, mp: 69-70°C; [α]_{D}²⁰ = - 56.6° (c = 1.0 in MeOH); MH⁺ = 647.

### D) Boc-D-TMSal-Pro-BoroLys-OPin

22.0 g (34.0 mmol) of the azide of Example 2, step C is dissolved in EtOAc and hydrogenated in the presence of 4.0 g of 10% Pd/C. After 9 h, the catalyst is removed and the solution is concentrated in vacuo. The resulting foam is dissolved in EtOAc and crystallized to give the desired Boc-TMSal-Pro-BoroLys-OPin as white crystals, mp: 128-129°C; [α]_{D}²⁰ = - 40.8° (c = 0.5 in CH₂Cl₂); MH⁺ = 621.

### E) Boc-D-TMSal-Pro-NH-CH[(CH₂)₄NHCHO]B-OPin

0.224 ml (5.95 mmol) Formic acid and 0.530 g (5.2 mmol) acetic anhydride are stirred 2 h at 60°C. The mixture is cooled to 0°C and 1.86 g (3.00 mmol) of the borolysine obtained in step D of Example 2 dissolved in THF is added. After 10 min stirring at 0°C, the reaction mixture is stirred at room temperature overnight. 80 ml of icewater is added, the solution extracted several times with ether and washed with brine. After drying over Na₂SO₄ and concentration in vacuo, the desired product is obtained as a white foam which can be crystallized from ether/hexane; mp: 93-99°C; [α]_{D}²⁰ =-77.8° (c = 0.5 in MeOH); MH⁺ = 649.

The Boc-D-TMSal-Pro-NH-CH[(CH₂)₅NHCHO]B-OPin([α]_{D}²⁰ = - 47.2° (c = 0.5 in MeOH); MH⁺ = 663) and Boc-D-TMSal-Pro-NH-CH[(CH₂)₃NHCHO]B-OPin ([α]_{D}²⁰ = - 68.0° (c = 0.5 in MeOH); MH⁺ = 635) products are obtained analogously.

### Example 5-14

### Boc-D-TMSal-Pro-NH-CH[(CH₂)₄CH₃]B-OPin

20 µl (0.25 mmol) Pyridine and 18 µl (0.25 mmol) acetyl chloride in CH₂Cl₂ are cooled to 0°C, 155.2 mg (0.25 mmol) of the borolysine product of Example 2, step D is added and the solution is stirred for 1 h. Water is added, the product extracted several times with ether, washed with brine, dried over Na₂SO₄ and concentrated in vacuo. The desired product is obtained as an oil; [α]_{D}²⁰ = - 62.6° (c = 0.5 in MeOH); MH⁺ = 663.

The following (Boc-D-TMSal-Pro-NH-CH[(CH₂)₄NHC(O)**R**]B-OPin) products wherein R is as indicated in the table below are prepared analogously from the corresponding acyl chlorides or bromides:

| Ex. | R | [α]_{D}²⁰ (c = 0.5 in MeOH) | MH⁺ |
|---|---|---|---|
| 6 | Et | - 55.8° | 667 |
| 7 | i-Pr | - 60.2° | 691 |
| 8 | CH₂F | - 58.8° | 681 |
| 9 | CH₂Br | - 58.8° | 741 |
| 10 | CH₂OMe | - 79.8° | 693 |
| 11 | CO₂Me | - 64.8° | 707 |
| 12 | CO₂Et | - 59.6 ° | 721 |
| 13 | NMe₂ | - 64 2° | 692 |
| 14 | OCH₂Cl | - 62.0° | 713 |

### Example 15-16

### Boc-D-TMSal-Pro-NH-CH[(CH₂)₄NHC(O)CH₂Cl]B-OPin

124.2 mg (0.2 mmol) of the borolysine product of Example 2, step D in THF is cooled to 0°C and 35.2 mg (0.2 mmol) monochloroacetic anhydrid is added. After stirring for 3 h at room temperature, the solvent is removed in vacuo and the product is obtained as a white foam; [α]_{D}²⁰ = - 57.8° (c = 0.5 in MeOH); MH⁺ = 697.

The Boc-D-TMSal-Pro-NH-CH[(CH₂)₄NHC(O)CCl₃]B-OPin product is prepared analogously; [α]_{D}²⁰ = - 54.0° (c = 0.5 in MeOH); MH⁺ = 765.

### Example 17

### Boc-D-TMSal-Pro-NH-CH[(CH₂)₄[NHC(O)OCH₃B-Opin

58.7 µl (0.24 mmol) N,O-Bis(trimethylsilyl)acetamide in CH₂Cl₂ is added to 124.2 mg (0.20 mmol) of the borolysine product of Example 2, step D in CH₂Cl₂ and stirred for 1 h at room temperature. After cooling to 0°C, 19.5 µl (0.24 mmol) methyl chloroformate in CH₂Cl₂ is added and the mixture stirred for 5 h at 0°C. The CH₂Cl₂ is removed and pH 7 buffer is added at 0°C. The product is extracted several times with ether, washed with brine, dried over Na₂SO₄ and concentrated in vacuo, yielding a white foam; [α]_{D}²⁰ = - 59.6° (c = 0.5 in MeOH); MH⁺ = 679.

### Example 18-22

### Boc-D-TMSal-Pro-NH-CH[(CH₂)₄NHC(S)NHCH₃]B-OPin

124.2 mg (0.2 mmol) of the borolysine product of example 2, step D in CH₂Cl₂ is cooled to 0°C and 15 mg (0.2 mmol) methyl isothiocyanate is added. After stirring 7 h at room temperature, the solvent is removed in vacuo and the product is obtained as a white foam, which can be crystallized from ether/hexane; mp: 109-113°C; [α]_{D}²⁰ = - 66.8° (c = 0.5 in MeOH); MH⁺ = 694.

Analogously Boc-D-TMSal-Pro-NH-CH[(CH₂)₄NHC(S)NHEt]B-OPin ([α]_{D}²⁰ = - 66.4° (c = 0.5 in MeOH); MH⁺ = 708), Boc-D-TMSal-Pro-NH-CH[(CH₂)₄NHC(O)NHCH₃]B-OPin ([α]_{D}²⁰ = - 62.2° (c = 0.5 in MeOH); MH⁺ = 678), Boc-D-TMSal-Pro-NH-CH[(CH₂)₄NHC(O)-NHEt]B-OPin ([α]_{D}²⁰ = - 65.6° (c = 0.5 in MeOH); MH⁺ = 692) and Boc-D-TMSal-Pro-NH-CH[(CH₂)₃NHC(O)NHEt]B-OPin ([α]_{D}²⁰ = - 71.0° (c = 0.5 in MeOH); MH⁺ = 678), are obtained from the corresponding iso(thio)cyanates.

### Example 23

### Boc-D-TMSal-Pro-NH-CH[(CH₂)₄NHCHS]B-OPin

217 mg (0.35 mmol) of the formamide product of Example 2, step E in toluene is treated with 70.8 mg (0.175 mmol) Laweson-Reagent. After strirring for 1.5 h at room temperature, toluene is removed in vacuo. The desired product is crystallized from ether; mp: 123-125°C; [α]_{D}²⁰ = - 74.8° (c = 0.5 in MeOH); MH⁺ = 665.

### Example 24

### Boc-D-TMSal-Pro-NH-CH[(CH₂)₄NHC(S)CH₃]B-OPin

124 mg (0.2 mmol) of the borolysine product of Example 2, step D is dissolved in MeOH and 23 µl (0.2 mmol) ethyl dithioacetate in MeOH is added at room temperature. After stirring for 2.5 h at 60°C, the solvent is removed in vacuo. The residue is purified by RP-chromatography (7:3, EtOH/H₂H) and the desired product is obtained as a white foam; [α]_{D}²⁰ = - 65.8° (c = 0.5 in MeOH); MH⁺ = 679.

### Example 25-26

### Boc-D-TMSal-Pro-NH-CH[(CH₂)₄NHC(O)NH₂]B-OPin

248.3 mg (0.4 mmol) of the borolysine product of Example 2, step D and 0.4 ml 1 N HCI in H₂O is warmed to 50°C. After 5 min, 33.2 mg (0.4 mmol) of potassium cyanate is added in small portions. After 6 h at 50°C water is added, the product extracted with EtOAc, dried over Na₂SO₄ and concentrated in vacuo. After flash chromatography (1:9, hexane/EtOAc), the desired product is obtained; [α]_{D}²⁰ = - 71.7° (c = 0.5 in MeOH); MH⁺ = 664.

The Boc-D-TMSal-Pro-NH-CH[(CH₂)₃NHC(O)NH₂]B-OPin product is prepared analogously; [α]_{D}²⁰ = - 54.0° (c = 0.5 in MeOH); MH⁺ = 765.

### Example 27

### Boc-D-TMSal-Pro-NH-CH[(CH₂)₄NHC(O)CH₂OH]B-OPin

48 µl (0.6 mmol) pyridine and 99.6 µl (0.6 mmol) benzyloxyacetyl chloride in THF are cooled to 0°C and 372.4 mg (0.6 mmol) of the borolysine product of Example 2, step D is added. After 3 h at 0°C, water is added, the product extracted several times with ether, dried over Na₂SO₄ and concentrated in vacuo.

The resulting white foam (370 mg, 0.48 mmol) is dissolved in EtOH and hydrogenated in the presence of 0.5 g of 10% Pd/C at 40 psi. After 12 h, the catalyst is removed and the solution is concentrated in vacuo. After flash chromatography (9:1, EtOAC/EtOH), the desired product is obtained; [α]_{D}²⁰ = - 67.0° (c = 0.5 in MeOH); MH⁺ = 679.

### Example 28

### Boc-D-TMSal-Pro-NH-CH[(CH₂)₄NHSO₂CH₃]B-OPin

19.8 µl (0.25 mmol) methane sulfonic acid chloride in THF at 0°C is added to 20 µl (0.25 mmol) pyridine. 155.2 mg (0.25 mmol) of the borolysine product of Example 2, step D in THF is added to this mixture. After stirring for 2.5 h at 0°C, water is added, the product extracted with ether, dried over Na₂SO₄ and concentrated in vacuo, yielding the product as a white foam; [α]_{D}²⁰ = - 61.6° (c = 0.5 in MeOH); MH⁺ = 699.

### Example 29-31

### Boc-D-TMSal-Pro-NH-CH[(CH₂)₂C₆H₅]B-OPin

### A. (+)-Pinanediol-(S)-1-chloro-3-phenyl-propane-1-boronate

3.47 g (30.0 mmol) Styrene and 3.6 g (30.0 mmol) catecholborane are stirred for 20 h at 100°C, yielding a yellow-brown oil. This oil is added to 5.0 g (30.0 mmol) (+)- pinanediol in THF and stirred overnight. After flash chromatography (8:2, hexane/EtOAc) (+)-pinanediol-2-phenyl-ethane-1-boronate is obtained.

Over a 20 min period 15 ml (24.0 mmol) BuLi (1.6 M in hexane) are added to a precooled (-100°C) solution of 2.1 ml CH₂Cl₂ and 37 ml THF. After stirring for 30 min at -100°C, 6.2 g (21.8 mmol) of the pinanediolboronate product obtained above in THF is added over a 20 min period. The reaction mixture is again stirred for 1 h at -100°C, after which 1.51 g (10.9 mmol) ZnCl₂ in THF is added. The reaction mixture is allowed to warm up to room temperature overnight. After concentration in vacuo the residue is dissolved in ether/H₂O. The organic layer is dried over Na₂SO₄ and concentrated in vacuo. (+)-pinanediol-(*S*)-1-chloro-3-phenyl-propane-1-boronate is obtained as yellow-orange oil.

### B. Boc-D-TMSal-Pro-NH-CH[(CH₂)₂C₆H₅]B-OPin

5.1 ml (8.09 mmol) Buthyllithium (1.6 M in hexane) is added to a precooled ( - 78°C) solution of 1.7 ml (8.09 mmol) hexamethyldisilazane in THF. The reaction mixture is stirred 1 h at room temperature, then cooled down to -78°C again. 2.68 g (8.09 mmol) (+)-pinanediol-(S)-1-chloro-3-phenyl-propane-1-boronate dissolved in THF is added. After stirring for 1 h at -78°C, the solution is warmed up overnight to room temperature. After recooling to -78°C 3 mol equivalents of HCI in dioxane are added. The mixture is stirred for 1 h at -78°C, then for 2 h at room temperature. After cooling down to -20°C, a solution of 3.7 g (8.09 mmol) of the active ester of Example 1, step B in CH₂Cl₂ is added, followed by the addition of 2.3 ml (18.2 mmol) triethylamine. The mixture is stirred for 1 h at -20°C and for 2 h at room temperature, then filtered. After concentration in vacuo, the residue is dissolved in ether/H₂O. The organic layer is dried over Na₂SO₄ and concentrated in vacuo. After flash chromatography (6:4, hexane/EtOAc), the desired product is obtained; [α]_{D}²⁰ = - 88.6° (c = 0.5 in MeOH); MH⁺ = 654.

The Boc-D-TMSal-Pro-NH-CH[(CH₂)₂(*p*-Cl-C₆H₄)]B-OPin ([α]_{D}²⁰ = - 83.2° (c = 0.5 in MeOH); MH⁺ = 688) and Boc-D-TMSal-Pro-NH-CH[(CH₂)₂(*p*-NMe₂-C₆H₄]B-OPin ([α]_{D}²⁰ = - 84.8° (c = 0.54 in MeOH); MH⁺ = 697) products are obtained analogously.

### Example 32

### CH₃[O(CH₂)₂]₃OCH₂C(O)-D-TMSal-Pro-NH-CH[(CH₂)₄NHCHO]B-OPin

### A. CH₃[O(CH₂)₂]₃OCH₂C(O)-D-TMSal-Pro-NH-CH[(CH₂)₄N₃]B-OPin

5.15 g (8.00 mmol) of the azide product of Example 2, step C is added to 40.0 ml of acetic acid/conc. HCl (9:1) and stirred for 75 min. After concentration in vacuo, HCl·H-D-TMSal-Pro-NH-CH[(CH₂)₄N₃]B-OPin is crystallized from ether; mp: 87°C.

0.29 ml (2.40 mmol) pivaloyl chloride in THF is added to a mixture of 1.17 g (2.00 mmol) of the above abtained product and 0.34 ml (2.40 mmol) triethylamine in THF at 0°C and stirred for 1 h at this temperature. To this solution 533 mg (2.40 mmol) CH₃[O(CH)₂]₃OCH₂CO₂H in THF and subsequently 4.0 ml of saturated aqueous NaHSO₄ are added. After stirring for 1 h at room temperature, saturated aqueous NaHSO₄ is added and the product is extracted several times with EtOAc. The organic layers are washed with 2 N H₂SO₄ and brine, dried over Na₂SO₄ and concentrated in vacuo. The crude product is dissolved in MeOH/water (6:4) and washed with hexane. After concentration of the MeOH/water layer, CH₃[O(CH₂)₂]₃OCH₂C(O)-D-TMSal-Pro-NH-CH[(CH₂)₄N₃]B-OPin is obtained as an oil; [α]_{D}²⁰ = - 74.5° (c = 0.83 in MeOH); MH⁺ = 751.

### B. CH₃[O(CH₂)₂]₃OCH₂C(O)-D-TMSal-Pro-NH-CH[(CH₂)₄NH₂]B-OPin

1.05 g (1.40 mmol) of the product of Example 32, step A is disolved in 14 ml EtOH and hydrogenated in the presence of 140 mg of 10% Pd/C and 0.84 ml (1.38 mmol) 2 N HCl. After 2.5 h, the catalyst is removed and water is added. The water layer is washed with other and concentrated in vacuo, yielding 855 mg (80 %) CH₃[O(CH₂)₂]₃OCH₂C(O)-D-TMSal-Pro-NH-CH[(CH₂)₄NH₂]B-OPin as an oil [α]_{D}²⁰ = - 74.2° (c = 0.61 in MeOH); MH⁺ = 725.

### C. CH₃[O(CH₂)₂]₃OCH₂C(O)-D-TMSal-Pro-NH-CH[(CH₂)₄NHCHO]B-OPin

31 µl (0.80 mmol) formic acid and 71 µl (0.70 mmol) acetic anhydrid are stirred for 2 h at 50°C. After cooling to 0°C, 325 mg (0.43 mmol) of the borolysine product of Example 32, step B in THF and 0.12 ml (0.86 mmol) triethylamine is added. After stirring for 17 h at room temperature, EtOAc is added and the solution washed with 2 N H₂SO₄ and brine, dried over Na₂SO₄ and concentrated in vacuo. After RP-chromatography (MeOH/water, gradient 50% H₂O → 25 % H₂O), the desired product is obtained as an oil; [α]_{D}²⁰ = - 80.8° (c = 0.27 in MeOH); MH⁺ = 753.

### Example 33

### CH₃[O(CH₂)₂]₃OCH₂C(O)-D-TMSal-Pro-NH-CH[(CH₂)₄NHC(O)NHEt]B-OPin

325 mg (0.43 mmol) of the borolysine product of Example 32, step B in THF is cooled to 0°C and 40 µl (0.43 mmol) ethyl isocyanate and 48 µl (0.43 mmol) N-methyl morpholine is added. After 4 h stirring at room temperature, EtOAc is added and the solution washed with 1 N NaHSO₄, saturated aqueous NaHCO₃ and brine, dried over Na₂SO₄ and concentrated in vacuo. After RP-chromatography (MeOH/water, gradient 50% H₂O → 25 % H₂O), the desired product is obtained as an oil; [α]_{D}²⁰ = - 76.0° (c = 0.55 in MeOH); MH⁺ = 796.

### Example 34

### H-D-TMSal-Pro-NH-CH[(CH₂)₄NHCHO]B(OH)₂

150 mg (0.231 mmol) of the formamide product of Example 2, step E is dissolved in 2.5 ml acetic acid/conc. HCl (1:9) and stirred fcr 4 h at room temperature. After concentration in vacuo, the residue is dissolved 3 times in toluene and again concentrated in vacuo. The desired product is obtained as yellow foam; [α]_{D}²⁰ = - 63.6° (c = 0.55 in MeOH); MH⁺ = 415.

### Example 35

### H-D-TMSal-Pro-NH-CH[(CH₂)₄NHC(O)NHEt]B(OH)₂

150 mg (0.217 mmol) of the Boc-D-TMSal-Pro-NH-CH[(CH₂)₄NHC(O)NHEt]B-OPin product of Example 21 is dissolved in 2.5 ml acetic acid/conc. HCI (1:9) and stirred for 4 h at room temperature. After concentration in vacuo, the residue is dissolved 3 times in toluene and again concentrated in vacuo. The desired product is obtained as orange foam; [α]_{D}²⁰ = - 74.1] ° (c = 0.56 in MeOH); MH⁺ = 458.

## Claims

1. A compound of formula I 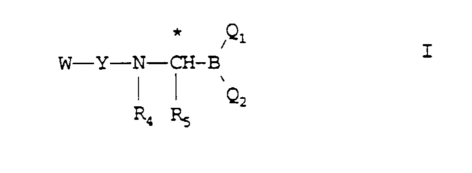 wherein: W is hydrogen or an N-protecting group
Y is a sequence of 2 amino acids of which the N-terminal amino acid is an unnatural amino acid having a hydrophobic side chain and the other amino acid is L-proline, the unnatural amino acid being selected such that the amino acid peptide Y-Lys or Y-Arg has an affinity for the active site of a trypsin-like protease;
Q₁ and Q₂ are the same or different and are selected from -OH, -COR₁, -CONR₁R₂, -NR₁R₂ or -OR₃ or Q₁ and Q₂ taken together form a diol residue;
R₁, R₂ and R₃ which may be the same or different, are C₁₋₁₀alkyl, C₆₋₁₀aryl, C₆₋₁₀ aralkyl or phenyl substituted by up to three groups selected from C₁₋₄alkyl halogen and C₁₋₄alkoxy;
R₄ is hydrogen or C₁₋₁₀alkyl
R₅ is a group -A-X
wherein A is -(CH₂)_{z}- in which z is 2,3,4 or 5; -CH(CH₃)-(CH₂)₂-; -CH₂-CH(CH₃)-CH₂-; -(CH₂)₂-CH(CH₃)-; -(CH₂)₂-C(CH₃)₂-; -CH(CH₃)-(CH₂)₃-; -CH₂-CH(CH₃)-(CH₂)₂-; -CH₂-CH₂-CH(CH₃)-CH₂; -(CH₂)₂C(CH₃)₂-CH₂-; (CH₂)₃-CH(CH₃)-; -(CH₂)₃-C(CH₃)₂: -(CH₂)₃CH(CH₃)CH₂-; C₆₋₁₀aryl, C₆₋₁₀aralkyl
and wherein X is OH, SH, NR₆R₇ or phenyl,
wherein R₆ is H or C₁₋₁₀alkyl, and R₇ is C₁₋₁₀alkyl -CO-R₈, CS-R₈, or SO₂-R₈
wherein R₈ is H or C₁₋₁₀alkyl, C₁₋₁₀alkoxy, C₆₋₁₀aryl, C₆₋₁₀aralkyl optionally substituted by up to three groups selected from halogen, OH, C₁₋₄alkoxy, C(NH)R₉; NR₁₀R₁₁ and C(O)OR₆ (wherein R₆ is as defined above)
wherein R₉ is C₁₋₃alkyl or N(CH₃)₂ and wherein R₁₀ and R₁₁ (which may be the same or different) are H or C₁₋₁₀alkyl
and the asymmetric carbon atom marked ∗ may have the D- or L-configuration, and the compounds may comprise any mixture of these.

2. A compound according to claim 1 in which W is H(CH₂CH₂0)ₚ-, R₁₂C0-, R₁₃OCO- or R₁₄SO₂-, wherein:
R₁₂ = C₁₋₆alkyl or H(CH₂-O-CH₂)ₚ
R₁₃ = C₁₋₆alkyl, phenyl, benzyl or naphthyl; and
R₁₄ = phenyl, naphthyl or C₁₋₄alkylphenyl
wherein p = 3-30

3. A compound according to claim 1 or 2 which is of formula Ia 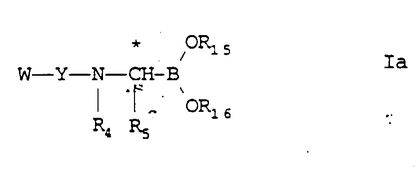 wherein: W,Y,R₄ and R₅ are as defined in claims 1 or 2 and R₁₅ and R₁₆ represent the residue of a dihydroxy compound.

4. A compound according to claim 1 or 2 wherein Q₁ and Q₂ together represent a group of formula (a) or (b) 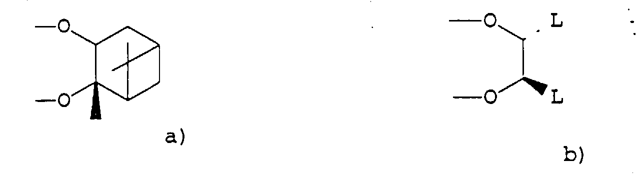 in which L is a C₁₋₄alkyl group.

5. A compound according to any one of claims 1 to 4 wherein the unnatural amino acid is of formula II 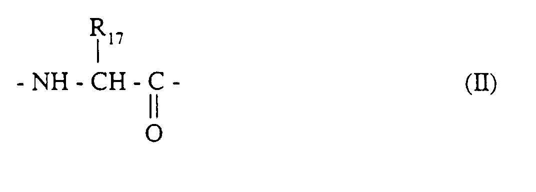 wherein R₁₇ is a hydrophobic group consisting of an alicyclic group having at least two rings and no polar substituents linked to the amino acid either directly or through a methylene group; a methylene group linked to an aromatic group optionally substituted by a polar group, or to a tert. butyl or tri-methylsilyl group.

6. A compound according to claim 5 wherein R₁₇ is R₁₇' and is a group of formula (c), (d), (e), (f), (g), (h) or (i)

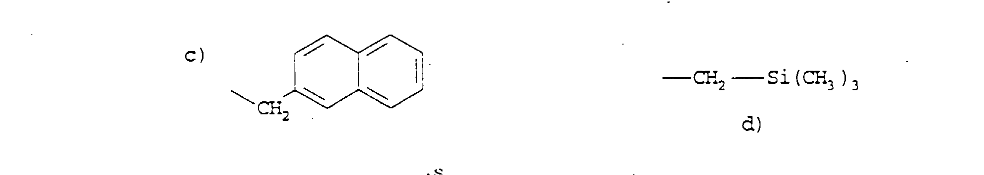

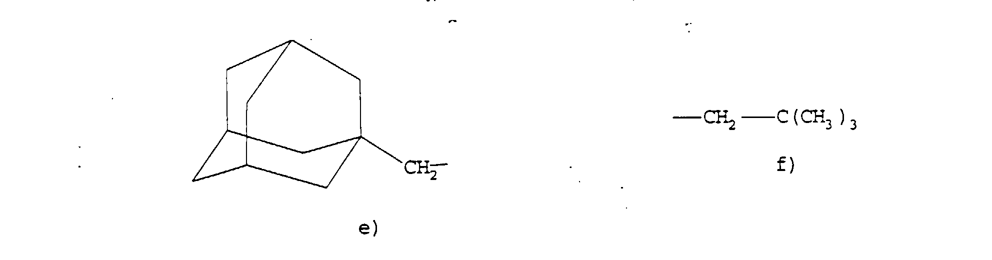

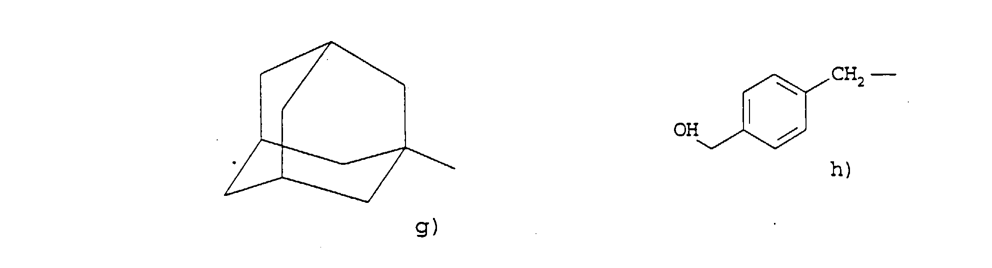

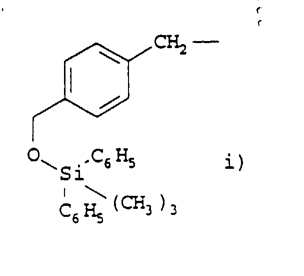

7. A compound according to claim 1 selected from the compounds of formulae III, IV, V and VI

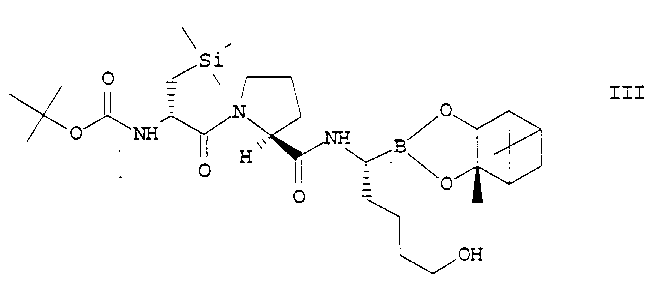

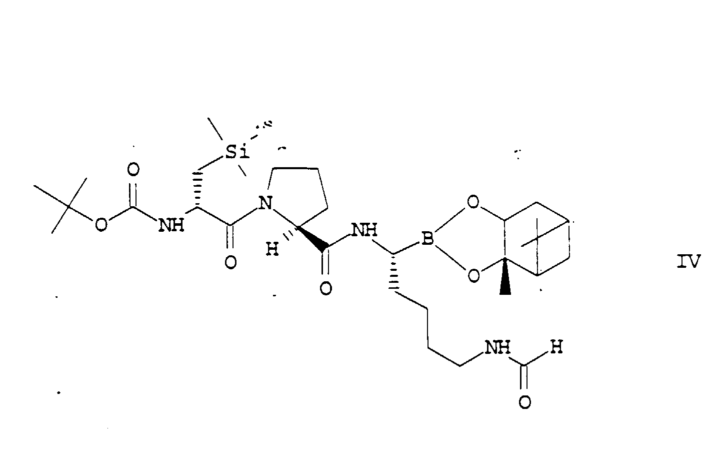

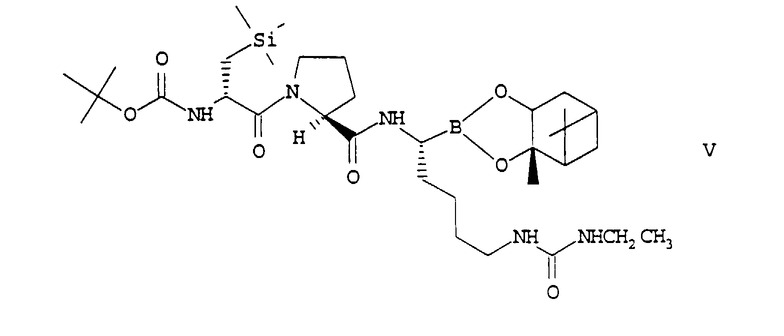

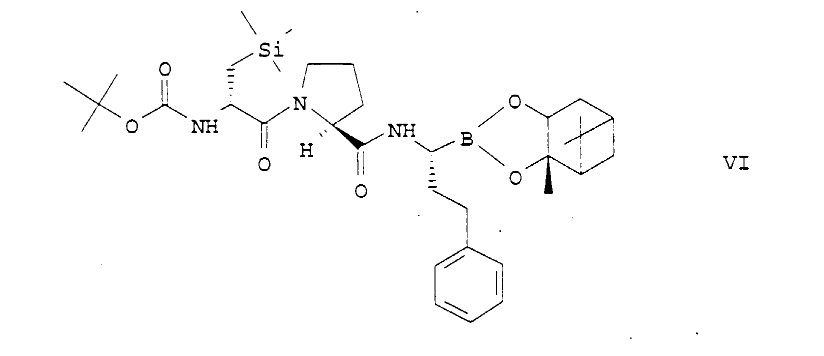

8. A process for the preparation of a compound of formula I as defined in claim 1 which comprises:
i) when X is NHCHO or NHCOalkyl, reacting a compound of formula I in which X is -NH₂ with an active form of the corresponding acid;
ii) when X is NHCOalkoxy, treating the compound of formula I in which X is -NH₂ with N, O -bis (trimethylsilyl) acetamide followed by addition of an active form of the corresponding ester;
iii) when X is NHC(S)NHalkyl, reacting the compound of formula I in which X is -NH₂ with the corresponding alkyl isothiocyanate;
iv) when X is NHCHS, reacting the compound of formula I in which X is -NHCHO with Laweson reagent;
v) when X is optionally substituted phenyl, reacting a compound of formulae VII 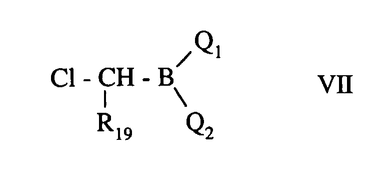 in which Q1 and Q2 are as defined in claim 1 and R₁₉ is -A-phenyl (optionally substituted) wherein A is as defined in claim 1, with LiN[Si(CH₃)₃]₂, followed by hydrolysis with an excess of acid and coupling with a protected peptide of formula VIII 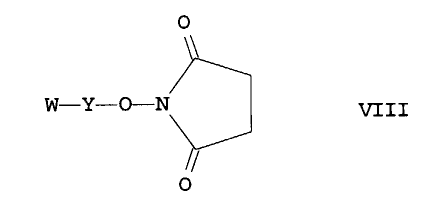 wherein W and Y are as defined in claim 1.
vi) when X is OH reacting a compound of formula I in which X is -OSi(CH₃)₂ C(CH₃)₃ with a desilylating agent.

9. A compound of any one of claims 1 to 7 for use as a pharmaceutical.

10. Therapeutic composition containing a compound of any one of claims 1 to 7 together with pharmaceutically acceptable additives and/or diluents.

11. Use of a compound of any one of claims 1 to 7 for the preparation of a medicament for inhibition of trypsin like serine proteases.

12. Use according to claim 11 characterised in that the trypsin-like serine protease is thrombin, factor Xₐ, kallikrein, plasmin, prolyl endopeptidase or Ig AI protease.

13. Therapeutic composition having anti-coagulant or anti-thrombogenic activity containing a compound of any one of claims 1 to 7 together with pharmaceutically acceptable additives and/or diluents.

14. Use according to claim 12 for inhibition of thrombin.

15. Use of a compound of any one of claims 1 to 7 for the preparation of a medicament for use as an inhibitor of vascular remodelling following venous or arterial surgery or other forms of vascular damage.

## Patentansprüche

1. Verbindung der Formel I 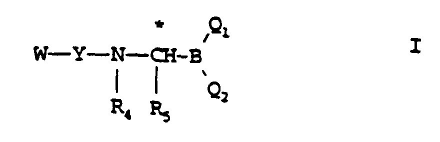 worin W für Wasserstoff oder eine N-Schutzgruppe steht,
Y für eine Sequenz aus 2 Aminosäuren steht, von denen die N-terminale Aminosäure eine unnatürliche Aminosäure mit einer hydrophoben Seitenkette ist und die andere Aminosäure L-Prolin ist, die unnatürliche Aminosäure so gewählt ist, daß das Aminosäurepeptid Y-Lys oder Y-Arg eine Affinität für das aktive Zentrum einer Trypsin-ähnlichen Protease aufweist,
Q₁ und Q₂ gleich oder verschieden sind und ausgewählt sind aus -OH, -COR₁, -CONR₁R₂, -NR₁R₂ oder -OR₃ oder Q₁ und Q₂ zusammengenommen einen Diolrest bilden,
R₁, R₂ und R₃, die gleich oder verschieden sein können, stehen für C₁₋₁₀Alkyl, C₆₋₁₀Aryl, C₆₋₁₀Aralkyl oder Phenyl, das mit bis zu drei Gruppen substituiert ist, die ausgewählt sind aus C₁₋₄Alkyl, Halogen und C₁₋₄Alkoxy,
R₄ für Wasserstoff oder C₁₋₁₀Alkyl steht,
R₅ für eine Gruppe -A-X steht,
worin A steht für (CH₂)_{z}-, worin z für 2, 3, 4 oder 5 steht, -CH(CH₃)-(CH₂)₂-, -CH₂-CH(CH₃)-CH₂-, -(CH₂)₂-CH(CH₃), -(CH₂)₂-C(CH₃)₂-, -CH(CH₃)-(CH₂)₃-, -CH₂-CH(CH₃(CH₂)₂-, -CH₂-CH₂-CH(CH₃-CH₂-, -(CH₂)₂C(CH₃)₂-CH₂-, -(CH₂)₃-CH(CH₃)-, -(CH₂)₃-C(CH₃)₂-, -(CH₂)₃CH(CH₃)CH₂-, C₆₋₁₀Aryl, C₆₋₁₀Aralkyl, und worin X für OH, SH, NR₆R₇ oder Phenyl steht,
worin R₆ für H oder C₁₋₁₀Alkyl steht und R₇ für C₁₋₁₀Alkyl, -CO-R₈, CS-R₈ oder SO₂-R₈ steht,
worin R₈ steht für H oder C₁₋₁₀Alkyl, C₁₋₁₀Alkoxy, C₆₋₁₀Aryl, C₆₋₁₀Aralkyl, das wahlweise mit bis zu drei Gruppen subtituiert ist, die ausgewählt sind aus Halogen, OH, C₁₋₄Alkoxy, C(NH)R₉, NR₁₀R₁₁ und C(O)OR₆ (worin R₆ wie oben definiert ist)
worin R₉ für C₁₋₃Alkyl oder N(CH₃)₂ steht und worin R₁₀ und R₁₁ (die gleich oder verschieden sein können) für H oder C₁₋₁₀Alkyl stehen,
und das mit ∗ markierte asymmetrische Kohlenstoffatom die D- oder L-Konfiguration aufweist und die Verbindungen jedes Gemisch aus diesen enthalten können.

2. Verbindung nach Anspruch 1, worin W für H(CH₂CH₂O)ₚ-, R₁₂CO-, R₁₃OCO- oder R₁₄SO₂- steht, worin
R₁₂ für C₁₋₆Alkyl oder H(CH₂-O-CH₂)p steht
R₁₃ für C₁₋₆Alkyl, Phenyl, Benzyl oder Naphthyl steht, und
R₁₄ für Phenyl, Naphthyl oder C₁₋₄Alkylphenyl steht
worin p für 3-30 steht.

3. Verbindung nach Anspruch 1 oder 2 der Formel Ia 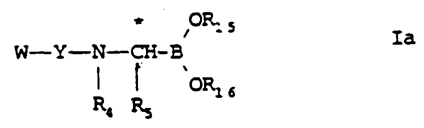 worin W, Y, R₄ und R₅ wie in den Ansprüchen 1 oder 2 definiert sind und R₁₅ und R₁₆ für den Rest einer Dihydroxyverbindung stehen.

4. Verbindung nach Anspruch 1 oder 2, worin Q₁ und Q₂ zusammen für eine Gruppe der Formel (a) oder (b) stehen 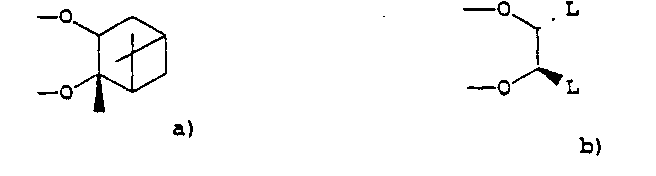 worin L für eine C₁₋₄Alkylgruppe steht.

5. Verbindung nach einem der Ansprüche 1 bis 4, worin die unnatürliche Aminosäure die Formel II aufweist 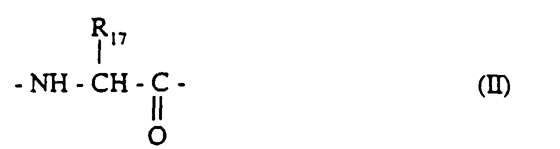 worin R₁₇ für eine hydrophobe Gruppe steht, die aus einer alicyclischen Gruppe mit mindestens zwei Ringen ohne polare Substituenten besteht und entweder direkt oder über eine Methylengruppe an die Aminosäure gebunden ist, wobei die Methylengruppe an eine aromatische Gruppe, die wahlweise mit einer polaren Gruppe substituiert ist, oder an eine tert-Butyl oder Trimethylsilylgruppe gebunden ist.

6. Verbindung nach Anspruch 5, worin R₁₇ für R₁₇' steht und eine Gruppe der Formel (c), (d), (e), (f), (g), (h) oder (i) ist

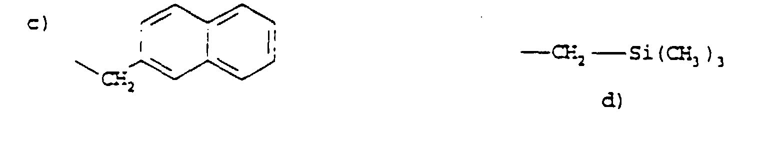

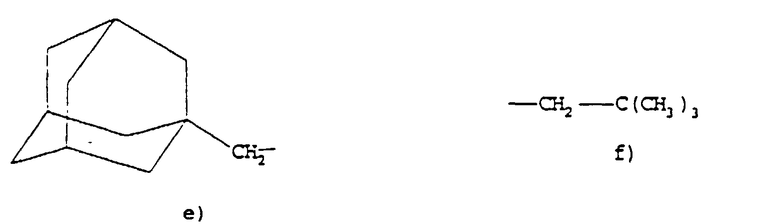

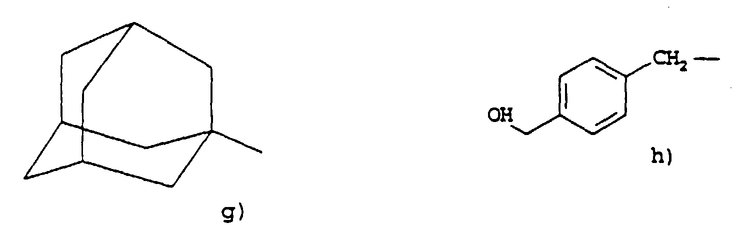

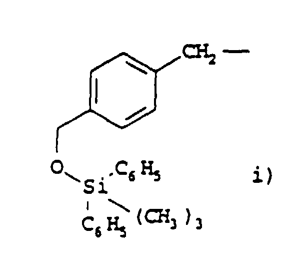

7. Verbindung nach Anspruch 1, die aus den Verbindungen der Formeln III, IV, V und VI ausgewählt ist

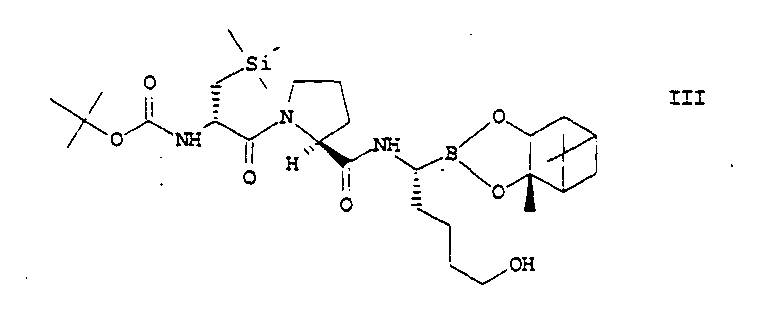

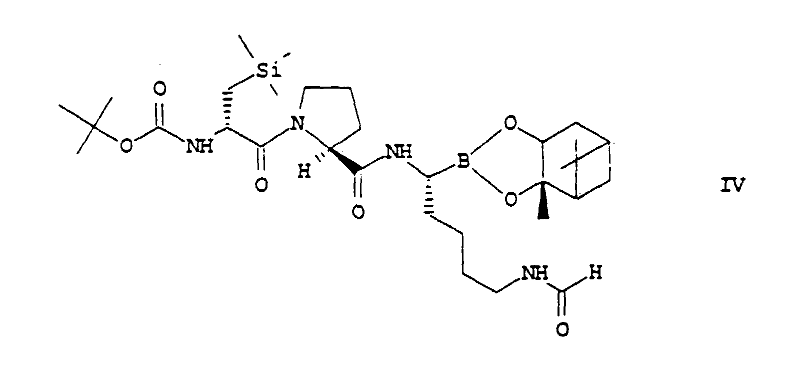

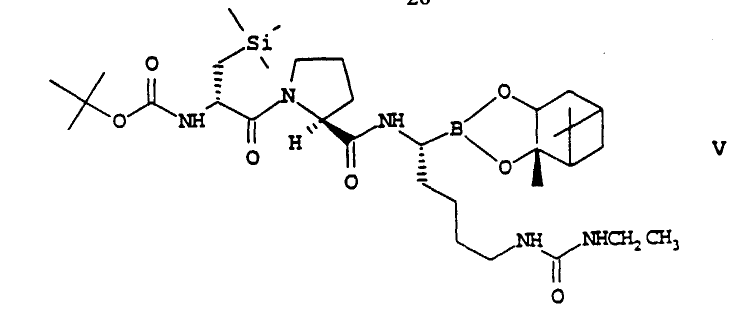

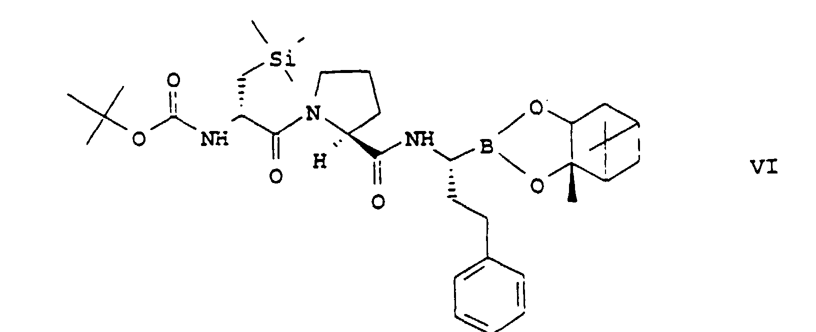

8. Verfahren zur Herstellung einer Verbindung der Formel I nach Anspruch 1, gekennzeichnet durch
(i) wenn X für NHCHO oder NHCOAlkyl steht, Umsetzung einer Verbindung der Formel I, worin X für -NH₂ steht, mit einer aktiven Form der entsprechenden Säure,
(ii) wenn X für NHCOAlkoxy steht, Behandlung der Verbindung der Formel I, worin X für -NH2 steht, mit N,O-Bis(trimethylsilyl)acetamid gefolgt von der Zugabe einer aktiven Form des entsprechenden Esters,
(iii) wenn X für NHC(S)NHAlkyl steht, Umsetzung der Verbindung der Formel I, worin X für -NH₂ steht, mit dem entsprechenden Alkylisothiocyanat,
(iv) wenn X für NHCHS steht, Umsetzung der Verbindung der Formel I, worin X für -NHCHO steht, mit einem Laweson-Reagenz,
(v) wenn X für ein wahlweise substituiertes Phenyl steht, Umsetzung einer Verbindung der Formel VII 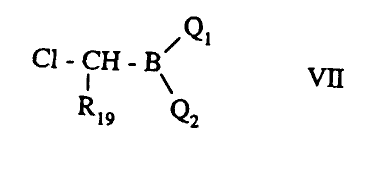 worin Q₁ und Q₂ nach Anspruch 1 definiert sind und R₁₉ für -A-Phenyl (wahlweise substituiert) steht, worin A nach Anspruch 1 definiert ist, mit LiN[Si(CH₃)₃]₂, gefolgt von der Hydrolyse mit einem Säureüberschuß und einer Kupplung mit einem geschützten Peptid der Formel VIII 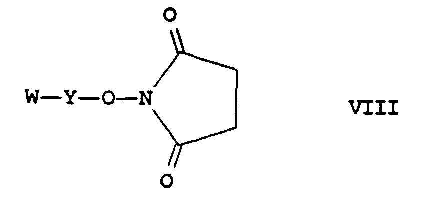 worin W und Y nach Anspruch 1 definiert sind, und
(vi) wenn X für OH steht, Umsetzung einer Verbindung der Formel I, worin X für -OSi(CH₃)₂-C(CH₃)₃ steht, mit einem Desilylierungsmittel.

9. Verbindung nach einem der Ansprüche 1 bis 7 zur Verwendung als Pharmazeutikum.

10. Therapeutische Zusammensetzung die eine Verbindung nach einem der Ansprüche 1 bis 7 zusammen mit pharmazeutisch annehmbaren Zusätzen und/oder Verdünnungsmitteln enthält.

11. Verwendung einer Verbindung nach einem der Ansprüche 1 bis 7 zur Herstellung eines Arzneimittels zur Hemmung von Trypsin-ähnlichen Serinproteasen.

12. Verwendung nach Anspruch 11, dadurch gekennzeichnet, daß die Trypsin-ähnliche Serinprotease Thrombin, Faktor Xa, Kallikrein, Plasmin, Prolylendopeptidase oder IgAI Protease ist.

13. Therapeutische Zusammensetzung mit einer antikoagulierenden oder antithrombogenen Aktivität, die eine Verbindung nach einem der Ansprüche 1 bis 7 zusammen mit pharmazeutisch annehmbaren Zusätzen und/oder Verdünnungsmitteln enthält.

14. Verwendung nach Anspruch 12 zur Hemmung von Thrombin.

15. Verwendung einer Verbindung nach einem der Ansprüche 1 bis 7 zur Herstellung eines Arzneimittels zur Verwendung als Inhibitor des vaskulären Wiederaufbaus nach einer venösen oder arteriellen Operation oder einer anderen Form der vaskulären Zerstörung.

## Revendications

1. Un composé de formule I 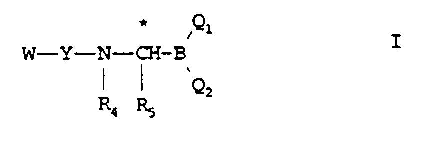 dans laquelle
W signifie l'hydrogène ou un groupe N-protecteur,
Y signifie une séquence de 2 aminoacides dont l'aminoacide N-terminal est un aminoacide non naturel ayant une chaîne latérale hydrophobe et l'autre aminoacide est la L-proline, l'aminoacide non naturel étant choisi afin que le peptide Y-Lys ou Y-Arg ait une affinité pour le site actif d'une protéase de type trypsine;
Q₁ et Q₂ sont identiques ou différents et sont choisis parmi -OH, -COR₁, -CONR₁R₂, -NR₁R₂ ou -OR₃ ou bien Q₁ et Q₂ pris ensemble forment un résidu diol;
R₁, R₂ et R₃ qui peuvent être identiques ou différents, signifient un groupe alkyle en C₁-C₁₀, aryle en C₆-C₁₀, aralkyle en C₆-C₁₀ ou phényle substitué par un maximum de 3 groupes choisis parmi les halogènes et les groupes alkyle en C₁-C₄, et alcoxy en C₁-C₄.
R₄ signifie l'hydrogène ou un groupe alkyle en C₁ -C₁₀,
R₅ signifie un groupe -A-X,
où A signifie -(CH₂)_{z}- dans lequel z signifie 2,3,4 ou 5; -CH(CH₃)-(CH₂)₂; -CH₂-CH(CH)₃-CH₂,; -(CH₂)₂-CH(CH₃)-; -(CH₂)₂-C(CH₃)₂-; -CH(CH₃)-(CH₂)₃-; -CH₂-CH(CH₃)-(CH₂)₂-; -CH₂-CH₂-CH(CH₃)-CH₂; -(CH₂)₂C(CH₃)₂-CH₂-; (CH₂)₃-CH(CH₃)-; -(CH₂)₃-C(CH₃)₂; -(CH₂)₃CH(CH₃)CH₂-; aryle en C₆₋ C₁₀ ou aralkyle en C₆₋C₁₀.
et où X signifie OH, SH, NR₆R₇ ou un groupe phényle,
où R₆ signifie H ou un groupe alkyle en C₁-C₁₀, et R₇ signifie un groupe alkyle en C₁-C₁₀, -COR₈, CS-R₈ ou SO₂-R₈
où R₈ signifie H ou un groupe alkyle en C₁-C₁₀, alcoxy en C₁-C₁₀, aryle en C₆-C₁₀ ou aralkyle en C₆-C₁₀. éventuellement substitués par un maximum de 3 groupes choisis parmi les halogènes, OH, alcoxy en C₁-C₄, C(NH)R₉; NR₁₀R₁₁ et C(O)OR₆ (où R₆ est tel que défini plus haut),
où R₉ signifie un groupe alkyle en C₁-C₃ ou N(CH₃)₂ et où R₁₀ et R₁₁ (qui peuvent être identiques ou différents) signifient H ou un groupe alkyle en C₁-C₁₀,
et l'atome de carbone asymétrique marqué d'un astérisque * peut avoir la configuration D ou L,
et un mélange de ces composés.

2. Un composé selon la revendication 1 dans lequel W signifie
H(CH₂CH₂O)ₚ-, R₁₂CO-, R₁₃OCO- ou R₁₄SO₂-, où:
R₁₂ = un groupe alkyle en C₁-C₆ ou H(CH₂-O-CH₂)ₚ,
R₁₃ = un groupe alkyle en C₁-C₆, phényle, benzyle ou naphtyle, et
R₁₄ = un groupe phényle, naphtyle ou (alkyl en C₁-C₄)phényle
où p= 3-30

3. Un composé selon la revendication 1 ou 2 qui répond à la formule Ia 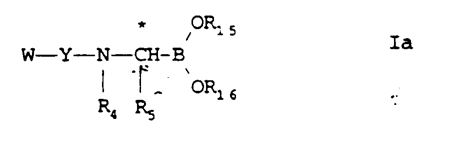 où W, Y, R₄ et R₅ sont tels que définis à la revendication 1 ou 2 et R₁₅ et R₁₆ signifient le résidu d'un composé dihydroxy.

4. Un composé selon la revendication 1 ou 2, où Q₁ et Q₂ signifient ensemble un groupe de formule (a) ou (b) 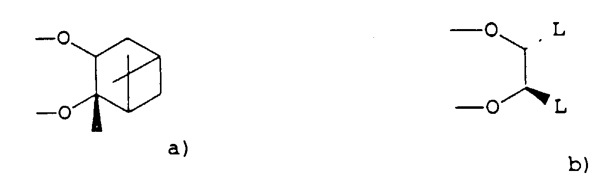 où L signifie un groupe alkyle en C₁-C₄.

5. Un composé selon l'une quelconque des revendications 1 à 4, dans lequel l'aminoacide non naturel est de formule II 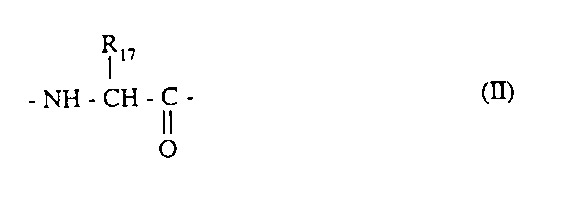 où R₁₇ signifie un groupe hydrophobe constitué d'un groupe alicyclique ayant au moins deux cycles et ne comportant aucun substituant polaire relié à l'aminoacide soit directement ou par un groupe méthylène; un groupe méthylène lié à un groupe aromatique éventuellement substitué par un groupe polaire ou lié à un groupe tert.-butyle ou tri-méthylsilyle.

6. Un composé selon la revendication 5, dans lequel R₁₇ signifie R₁₇' et signifie un groupe de formule (c), (d), (e), (f), (g), (h) ou (i)

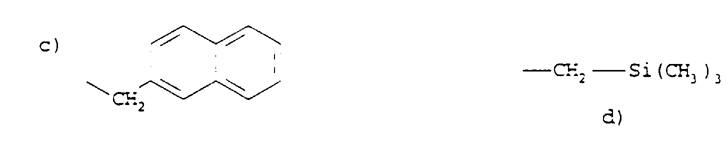

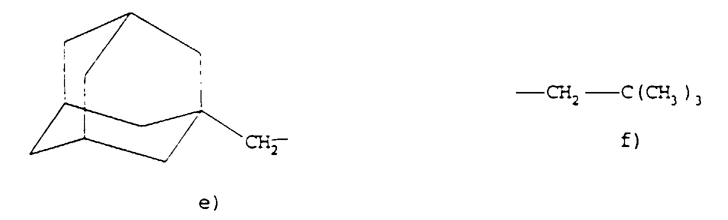

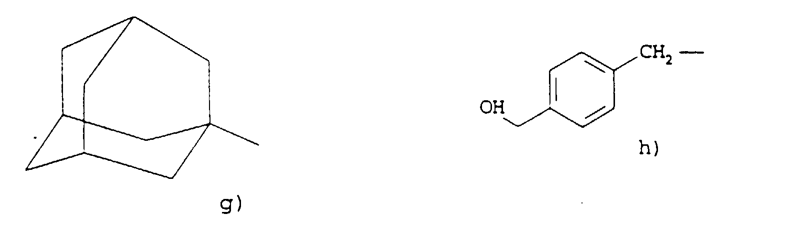

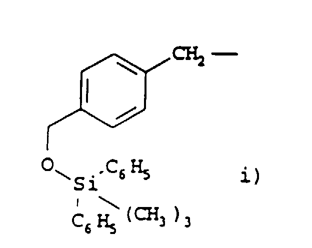

7. Un composé selon la revendication 1 choisi parmi les composés de formules III, IV, V et VI

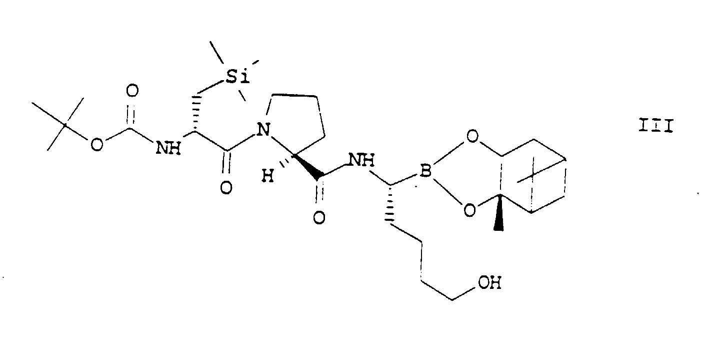

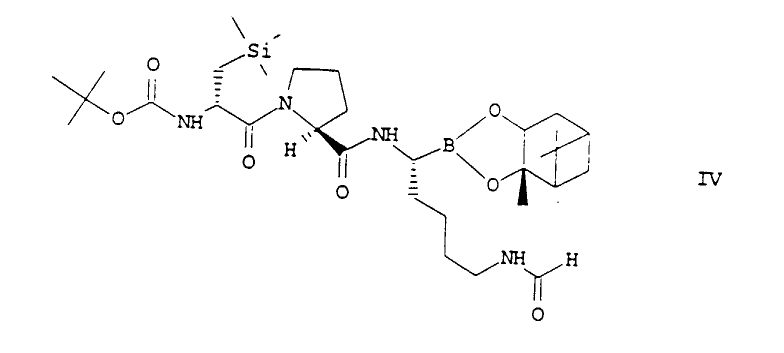

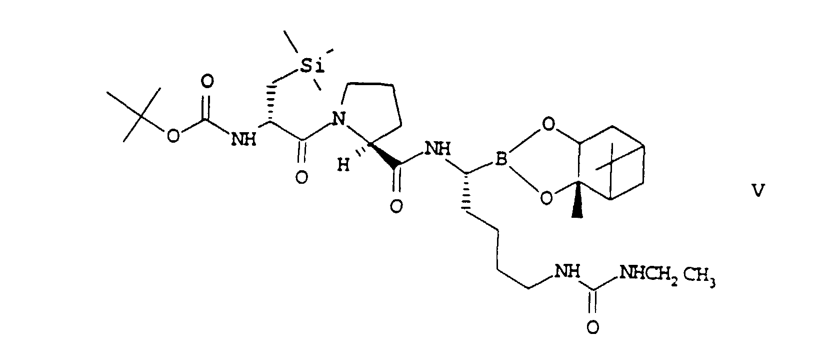

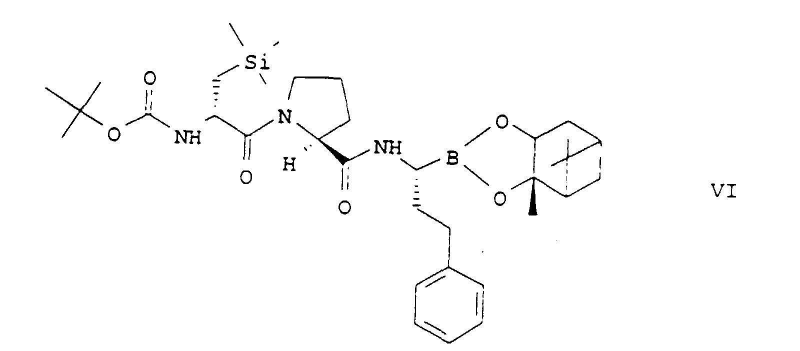

8. Un procédé de préparation d'un composé de formule I tel que défini à la revendication 1, selon lequel:
i) lorsque X signifie NHCHO ou NHCOalkyle, on fait réagir un composé de formule I où X signifie -NH₂, avec une forme active de l'acide correspondant;
ii) lorsque X signifie un groupe NHCOalcoxy, on fait réagir un composé de formule I où X signifie -NH₂, avec du N, O -bis(triméthylsilyl)-acétamide suivi par l'addition d'une forme active de l'ester correspondant;
iii) lorsque X signifie un groupe NHC(S)NHalkyle, on fait réagir un composé de formule I où X signifie -NH₂, avec un isothiocyanate d'alkyle correspondant;
iv) lorsque X signifie NHCHS, on fait réagir un composé de formule I où X signifie -NHCHO avec un réactif de Laweson;
v) lorsque X signifie un groupe phényle éventuellement substitué, on fait réagir un composé de formule VII 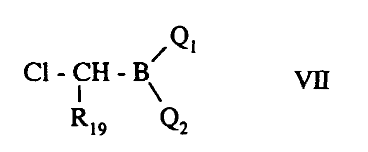 où Q₁ et Q₂ sont tels que définis à la revendication 1 et R₁₉ signifie un groupe -A-phényle (éventuellement substitué) où A est tel que défini à la revendication 1, avec LiN[Si(CH₃)₃]₂, puis on hydrolyse avec un excès d'acide et on couple avec un peptide protégé de formule VIII 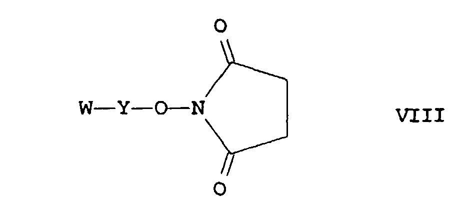 où W et Y sont tels que définis à la revendication 1;
vi) lorsque X signifie OH, on fait réagir un composé de formule I où X signifie -OSi(CH₃)₂C(CH₃)₃, avec un agent de désilylation.

9. Un composé selon l'une quelconque des revendications 1 à 7, pour une utilisation comme médicament.

10. Une composition thérapeutique contenant un composé selon l'une quelconque des revendications 1 à 7, en association avec des additifs et/ou des diluants pharmaceutiquement acceptables.

11. L'utilisation d'un composé selon l'une quelconque des revendications 1 à 7, pour la préparation d'un médicament pour l'inhibition des sérine-protéases de type trypsine.

12. L'utilisation selon la revendication 11, caractérisée en ce que la sérine-protéase de type trypsine est la thrombine, le facteur Xₐ, Ia kallicréine, la plasmine, la prolyl endopeptidase ou la protéase Ig AI.

13. Une composition thérapeutique ayant une activité anti-coagulante ou anti-thrombogène contenant un composé selon l'une quelconque des revendications 1 à 7, en association avec des additifs et/ou des diluants pharmaceutiquement acceptables.

14. L'utilisation selon la revendication 12, pour l'inhibition de la thrombine.

15. L'utilisation d'un composé selon l'une quelconque des revendications 1 à 7, pour la préparation d'un médicament pour l'utilisation comme inhibiteur du remodelage vasculaire suite à une opération chirurgicale des veines ou des artères ou à d'autres formes de lésions vasculaires.
